(19) Europäisches Patentamt
European Patent Office
Office européen des brevets

(11) Publication number: **0 073 732**
**B1**

(12) # EUROPEAN PATENT SPECIFICATION

(45) Date of publication of patent specification: **26.09.84**

(51) Int. Cl.³: **C 07 C149/20**, D 21 D 3/00, D 21 H 3/08

(21) Application number: **82810331.7**

(22) Date of filing: **06.08.82**

(54) **Ammonium and amine salts of di-perfluoroalkyl group containing acids, compositions and use thereof.**

(30) Priority: **12.08.81 US 292326**

(43) Date of publication of application:
**09.03.83 Bulletin 83/10**

(45) Publication of the grant of the patent:
**26.09.84 Bulletin 84/39**

(84) Designated contracting states:
**BE CH DE FR GB IT LI**

(56) References cited:
**DE-A- 2 753 128**

(73) Proprietor: **CIBA-GEIGY AG**
**Postfach**
**CH-4002 Basel (CH)**

(72) Inventor: **Falk, Robert A.**
**35 Glenside Drive**
**New City New York 10956 (US)**
Inventor: **Borsodi, Istvan**
**84 Alida Street**
**Yonkers, N.Y. 10704 (US)**
Inventor: **Reinehr, Dieter, Dr.**
**Wolfsheule 10**
**D-7842 Kandern (DE)**

**EP 0 073 732 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

**Description**

This invention relates to ammonium and amine salts of di-perfluoroalkyl group containing acids.

Di-perfluoroalkyl group containing acids, useful as starting materials in the manufacture of the ammonium and amine salts according to the present invention, are, in part, disclosed in US 4,239,915. The di-perfluoroalkyl group containing acids are disclosed therein as useful inter alia for textile treating or as chromium complexes which possess oil and water repellent properties and are indicated as useful as grease or oil repellents for paper. Unfortunately, these acids possess, in general, very limited water solubility and therefore require an organic solvent, or the like, for the application of the di-perfluoroalkyl group containing acids to the cellulose, synthetic or natural polyamide substrate in order to obtain the desired oil and water repellent properties. Such organic solvent containing solutions of the di-perfluoroalkyl group containing acids tend to be expensive due to the cost of such organic solvents. Moreover, the use of such organic solvent containing systems is often undesirable due to odor, toxicity and flammability hazards. Furthermore, such organic solvent containing systems tend to be inefficient since much of the fluorochemical tends to remain in the organic solvent system during the application process and must be subsequently recovered from the spent organic solvent liquor for re-use. Also environmental problems are usually inherent in the use or organic solvent systems on an industrial sclale. These problems can be overcome using new ammonium or amine salts of di-perfluoroalkyl group containing acids.

Thus, object of the present invention is to provide ammonium and amine salts of di-perfluoroalkyl group containing acids of the formula

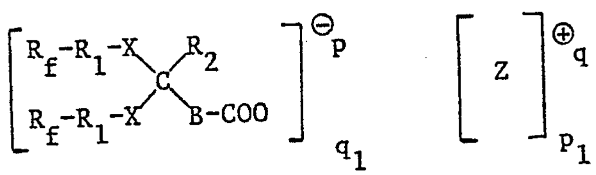

(1)

wherein

$R_f$ is perfluoroalkyl of 4 to 18 carbon atoms or perfluoroalkoxyperfluoroalkylene of 4 to 18 carbon atoms ;

$R_1$ is alkylene of 1 to 12 carbon atoms, alkylenethioalkylene of 2 to 12 carbon atoms, alkyleneoxyalkylene of 2 to 12 carbon atoms, or alkyleneiminoalkylene of 2 to 12 carbon atoms where the imino nitrogen atom contains as a third substituent, hydrogen or alkyl of 1 to 6 carbon atoms ;

X is —S—, —SO— or —$SO_2$— ;

$R_2$ is hydrogen, alkyl of 1 to 6 carbon atoms, phenyl, alkyl substituted phenyl of up to 18 carbon atoms, or is the carboxylic acid group —B—COOH, or

the carboxylate anion —B—COO ;

B is a covalent bond, or alkylene of up to 18 carbon atoms, phenylene, phenoxyalkylene of 7 to 14 carbon atoms, phenylalkylene of 8 to 14 carbon atoms, or phenylene substituted by alkyl of 1 to 4 carbon atoms ;

Z is the ammonium or amine cation ;

$^{\ominus}p$ is the anionic charge of the gem-perfluoroalkyl group containing acid and is 1 or 2,

$^{\oplus}q$ is the cationic charge of the ammonium or amine group Z and has a value of 1 to 200 ; and the cationic mole equivalent, $p_1$ is equal to $^{\ominus}p$ and the anion mole equivalent, $q_1$, is equal to $^{\oplus}q$, with the proviso that where $^{\ominus}p$ is 2 and $^{\oplus}q$ is an even integer, then $p_1$ is 1 and $q_1$ is $^{\oplus}q/2$.

It is a further object of the present invention to provide a process for the preparation of these salts.

Another object is to provide aqueous emulsion concentrates which contain these ammonium and amine salts.

Another object is to provide aqueous emulsions or dispersions containing these salts.

Another object is to provide a process for rendering oil and water repellent a cellulosic or natural or synthetic polyamide substrate.

Another object is to provide a process for the internal sizing of paper pulp to render the pulp oil and water repellent.

Another object is to provide cellulosic or natural or synthetic polyamide substrates which have been treated with the ammonium and amine salts according to the present invention.

These and other objects of the invention are apparent from the foregoing disclosure.

$R_f$ in the compounds of the formula (1) is a perfluorinated alkyl group. Suitable perfluoroalkyl groups contain 4 to 18, preferably 6 to 14 carbon atoms. Perfluoroalkyl groups having 6 to 12 carbon atoms are especially preferred. The perfluorinated alkyl groups $R_f$ can be derived from the corresponding alkyl radicals, e. g. butyl, 1-methylbutyl, hexyl, octyl, decyl, dodecyl, tetradecyl, hexadecyl or octadecyl. $R_f$ denotes further perfluoroalkylene. Such radicals $R_f$ contain preferably 4 to 18 carbon atoms or, more preferably, 5 to 12 carbon atoms and are of the formula $C_pF_{2p+1}$—O—$C_qF_{2p}$—, wherein the sum of p and q is an integer of from 4 to 18 or, preferably 5 to 12.

$R_1$ denotes an alkylene chain. Preferably, this chain contains 1 to 12 carbon atoms. Alkylene chains comprising 2 to 8 or, preferably, 2 to 4 carbon atoms such as ethylene, propylene or butylene are particularly preferred. $R_1$ denotes further alkylenethioalkylene, alkyleneoxyalkylene or alkyleneiminoalkylene, wherein the alkylene moieties contain 2 to 12 or, preferably 2 to 8 carbon atoms. Suitable alkylene groups are mentioned above. The nitrogen atom in the alkylene group is optionally substituted with alkyl having 1 to 6 carbon atoms such as methyl, ethyl, butyl or hexyl.

X is a linking group. Preferred are sulphur containing bivalent radicals such as —S—, —SO— or —$SO_2$—. —S— is mostly preferred.

$R_2$ denotes hydrogen or alkyl. Suitable alkyl groups may contain 1 to 6 carbon atoms such methyl, ethyl, propyl, butyl, pentyl or hexyl as well as branched isomers thereof. Preferred are those alkyl groups having 1 to 4 carbon atoms. Methyl and ethyl are especially preferred. Further, $R_2$ denotes an aromatic ring which is optionally substituted. Preferably, $R_2$ is an optionally substituted phenyl. Suitable substituents which may be present at the ring system are e. g. alkyl, preferably having 1 to 12 or, more preferably, 1 to 4 carbon atoms. $R_2$ further denotes the group —B—COOH or —B—$COO^{\ominus}$. In these formulae, B represents a covalent bond or a bivalent linking group. Such linking group may be, e. g., alkylene, optionally substituted phenylene, phenoxyalkylene or phenylalkylene. The alkylene linking groups contain preferably 1 to 18 carbon atoms. Alkylene chains having 1 to 6 carbon atoms are especially preferred. Most suitable are methylene, ethylene and propylene. The phenylene rings are optionally substituted by alkyl groups, which preferably contain 1 to 4 carbon atoms. The alkylene moieties in the phenoxyalkylene and phenylalkylene comprise preferably 1 to 8 and 2 to 8 carbon atoms, respectively. Suitable alkylene moieties are listed above.

Z is ammonium or represents an amine cation of the formula

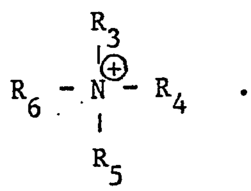

$R_3$ in this formula denotes hydrogen or alkyl, preferably having 1 to 5 carbon atoms such as methyl, ethyl, propyl, butyl or pentyl. The alkyl groups are optionally substituted and, in this case, contain preferably 2 to 6 or, more preferably, 1 to 3 carbon atoms. A preferred substituent for the alkyl groups is the hydroxy and carboxyl (COOH) group. Preferably, one carboxyl group or 2 to 6 hydroxy groups are present in a substituted alkyl group $R_3$. Further, $R_3$ denotes alkenyl having preferably 3 to 5 carbon atoms such as propylene, butylene, butadienyl and pentylene as well as other isomers thereof. If $R_3$ is cycloalkyl, rings having 5 to 7 carbon atoms are preferred. $R_3$ further denotes radicals containing ethyleneoxide units, preferably up to 30 units, or isopropyleneoxide units, preferably up to 6 units, or ethyleneoxide and isopropyleneoxide units. Suitable end groups for these units are e. g. hydrogen or alkyl, preferably having 1 to 3 carbon atoms. Further, $R_3$ is an aminoalkylene radical, wherein the alkylene moiety preferably contains 2 to 6 carbon atoms. The nitrogen atom in the aminoalkylene radicals is optionally substituted by alkyl groups, preferably having 1 to 3 carbon atoms or by radicals containing the above mentioned ethyleneoxide and isopropyleneoxide units. $R_3$ denotes further an alkylenediaminoalkylene group. Suitable alkyl moieties contain 2 to 8 carbon atoms. The (other) substituents on the nitrogen atoms are alkyl, preferably having 1 to 3 carbon atoms or alkyl substituted by hydroxy having preferably 2 to 6 carbon atoms. Another nitrogen substituent is represented by a chain containing the above mentioned ethyleneoxide and isopropyleneoxide units.

Instead of an alkylene linking group between the 2 nitrogen atoms of the alkylenediaminoalkylene radical, a cycloalkylene such as a cyclohexylene linking group may be used. $R_4$ and $R_5$ preferably are hydrogen, alkyl, preferably having 1 to 5 carbon atoms or an alkylene chain which contains said ethyleneoxide units. $R_4$ and $R_5$ may also form together with the nitrogen atom to which they are bonded a heterocyclic ring such as a morpholino ring. $R_6$ is an alkyl group, preferably having 1 to 5 carbon atoms. Together with $R_4$ and $R_5$, $R_6$ forms a unsaturated ring system such as a pyridyl radical. $R_6$ may denote hydrogen, if one of the other substituents $R_3$ to $R_5$ is different from hydrogen. If $R_3$ is different from hydrogen or alkyl, $R_6$ denotes an alkyl radical which preferably contains 6 to 23 carbon atoms. In addition to those alkyl radicals listed above, $R_6$ may denote nonadecyl, eicosyl, heneicosyl, docosyl or tricosyl as well as a branched isomer thereof. Further, $R_6$ denotes an alkylene chain which contains ethyleneoxide units.

The index $^{\ominus}p$ represents the anionic charge of the acid group and $^{\oplus}q$ denotes the cationic charge of the ammonium or amine group. The cationic mole equivalent $p_1$ is equal to $^{\ominus}p$ and the anion mole equivalent $q_1$ is equal to $^{\oplus}q$, with the proviso that where $^{\ominus}p$ is 2 and $^{\oplus}q$ is an even integer, then $p_1$ is 1 and $q_1$ is $^{\oplus}q/2$.

Suitable compounds of the formula (1) are those, wherein $R_f$ is perfluoroalkyl of 6 to 14 carbon atoms, $R_1$ is alkylene of 2 to 8 carbon atoms, X is —S— or —$SO_2$—, $R_2$ is hydrogen or alkyl of 1 to 4 carbon

atoms, —B— is a covalent bond or alkylene of 1 to 6 carbon atoms, $^{\ominus}$p is 1 and $^{\oplus}$q is 1 to 4. Of these compounds, those wherein X is —S— and q is 1 or 2 are especially preferred.

Further suitable compounds of the formula (1) are those, wherein $R_f$ is perfluoroalkyl of 6 to 12 carbon atoms, $R_1$ is alkylene of 2 to 4 carbon atoms, X is —S— or —$SO_2$—, $R_2$ is hydrogen or alkyl of 1 or 2 carbon atoms, —B— is a covalent bond or alkylene of 1 to 3 carbon atoms, $^{\ominus}$p is 1 and $^{\oplus}$p is 1 and $^{\oplus}$q is 1 or 2.

In another group of suitable compounds of the formula (1) Z is ammonium ($NH_4^{\oplus}$) or a water-soluble mono- or polyamino cation. Preferably, the water solubility of this mono- or polyamine cation at 25 °C is at least 2 % by weight in water.

Suitable mono- or polyamine cations correspond to the formula

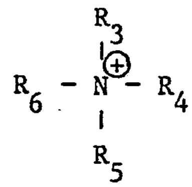 (2)

wherein $R_3$ is hydrogen, alkyl of 1 to 5 carbon atoms ; alkyl of 2 to 6 carbon atoms substituted by 2 to 6 hydroxy groups, alkenyl of 3 to 5 carbon atoms, cycloalkyl of 5 to 7 carbon atoms, —$C_1$—$C_3$— alkylene —COOH,

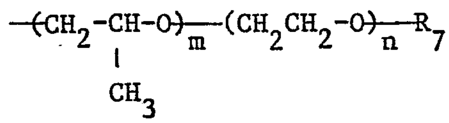

where m is 0 to 6, n is 0 to 30, and $R_7$ is hydrogen, alkyl of 1 to 3 carbon atoms or alkanoyl of 2 to 4 carbon atoms, or is

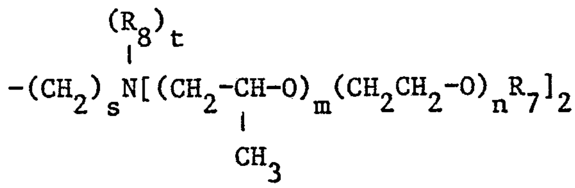

wherein s is 2 to 6, $R_8$ is hydrogen or alkyl of 1 to 3 carbon atoms, t is 0 or 1 and m, n and $R_7$ are as above defined ;

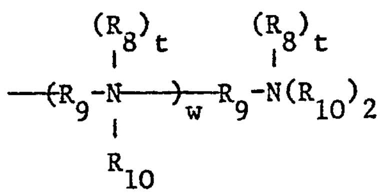

where $R_9$ is alkylene of 2 to 8 carbon atoms, cyclohexylene or di($C_1$ to $C_3$-alkylene) cyclohexylene, w is 0 to 198, $R_{10}$ is hydrogen, alkyl of 1 to 3 carbon atoms, alkyl of 2 to 6 carbon atoms substituted by hydroxy, or

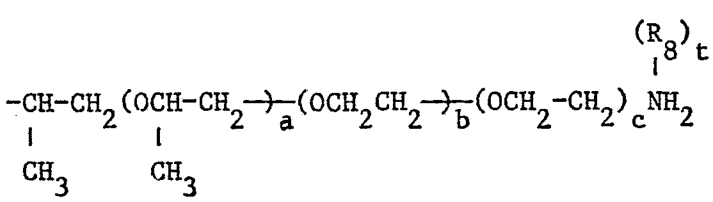

where a is 1 to 20, b is 2 to 50, c is 2 to 20 and $R_8$ and t are as defined above ;

$R_4$ and $R_5$ are independently hydrogen, alkyl of 1 to 5 carbon atoms or

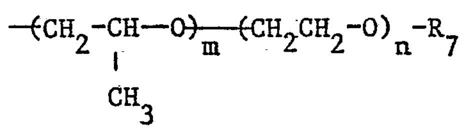

where m, n and $R_7$ are as defined above ; or

$R_4$ and $R_5$ taken together with the nitrogen to which they are attached represent morpholino ; and

$R_6$ is alkyl of 1 to 5 carbon atoms ; hydrogen when at least one of $R_3$, $R_4$ and $R_5$ is other than hydrogen ; alkyl of 6 to 23 carbon atoms when $R_3$ is other than hydrogen or alkyl of 1 to 5 carbon atoms ;

$$-(\underset{\displaystyle CH_3}{\underset{|}{CH}}-CH_2-O)_m-(CH_2CH_2-O)_nR_7$$

where m, n and $R_7$ are as defined above ;

or $R_4$, $R_5$ and $R_6$, taken together with the nitrogen to which they are attached represent pyridyl ; with the proviso that the total value of t equals $^{\oplus}q-1$.

In suitable mono- or polyamino cations of the formula (2) $R_3$ is $(CH_2CH_2—O)_n—H$ where n is 1 to 10, $R_4$ and $R_5$ are independently hydrogen or $(CH_2CH_2—O)_n—H$ where n is 1 to 10 and $R_6$ is hydrogen, or $(CH_2CH_2—O)_n—H$ where n is 1 to 10. Most preferably, n is 1 in each case. More preferably, in the compounds of the formula (2) $R_3$ is $—CH_2CH_2OH$, $R_4$ and $R_5$ are independently hydrogen or $—CH_2CH_2OH$, and $R_6$ is hydrogen or $—CH_2CH_2OH$.

In a further suitable group of compounds of the formula (2) $R_3$ is of the formula $(CH_2CH_2—O)_n—H$ where n is 1 to 10, $R_4$ is $(CH_2CH_2—O)_n—H$ where n is 1 to 10, $R_5$ is hydrogen or $(CH_2CH_2—O)_n—H$ where n is 1 to 10 and $R_6$ is alkyl of 6 to 20 carbon atoms. Most preferably, in this embodiment, the total value of n should be greater than 3 in order to insure the desired solubility of the amine.

Advantageously, the solubility of the amine in water is determined on the basis of free amine in water, or in the case of quaternary ammonium compounds, as the quaternary chloride.

The ammonium and amine salts according to the present invention are conventionally prepared by reacting the corresponding di-perfluoroalkyl group containing acids with ammonia or the amine in a diluent substantially inert to the acid and ammonia or amine reactants, and recovering the amine salt formed. As the reaction between the ammonia or amine and the acid takes place spontaneously, the reaction can be conducted at temperatures between 0 °C and 100 °C, preferably at ambient temperature conditions. Where the amine is introduced in gaseous form, such as anhydrous ammonia or methyl amine, it can be bubbled through the acid in the liquid diluent medium. As the reaction tends to be exothermic, cooling of the reaction vessel may be advantageously employed. Where the inert diluent is organic in nature, such as a lower alkanol, for example methanol, isopropanol, or the like, the ammonium or amine salt reaction product can be recovered by precipitation, or evaporation of the diluent. If the inert diluent is water, the ammonium or amine salt need not be separated from the aqueous media.

Where the amine is a quaternary ammonium compound, the resulting salt may be obtained by adding together in the inert diluent, such as water, the di-perfluoroalkyl group containing acid with the quaternary ammonium compound in the base, or hydroxide form. Alternatively, the di-perfluoroalkyl group containing acid may be neutralized with alkali or alkaline earth metal base, and the metal salt reacted with the quaternary ammonium compound in the halide form in an inert diluent by double displacement and the inorganic salt optionally separated therefrom by known extraction or precipitation techniques.

In preparing the instant amine salts from polyamines, one may utilize one or more equivalents of gem-perfluoroalkyl group containing acid per mole of polyamine. Thus, where Z in formula (1) is a polyamine, one or more amine nitrogens may be protonated, corresponding to the cationic charge, $^{\oplus}q$. In the preferred embodiment of the amine cation in formula (2), therefore, each additional protonated amine nitrogen, other than the nitrogen directly bonded to $R_3$, $R_4$, $R_5$ and $R_6$, is represented by the presence of an $R_8$ group, i. e. where t equals 1 as to such amine nitrogen. Accordingly, the total number of protonated amine nitrogens is $^{\oplus}q$, and the value of t equals $^{\oplus}q-1$. Similarly, the value of $^{\ominus}p$, corresponding to the anionic charge in formula (1), is 2 when $R_2$ is the carboxylate anion $—B—COO^{\ominus}$, and is 1 where $R_2$ is other than $—B—COO^{\ominus}$.

The cation mole equivalent in formula (1), $p_1$, is equal to the charge of its counter ion, i. e. the anionic ahrge $^{\ominus}p$, and the anion mole equivalent $q_1$ is equal to the charge of its counter ion, the cationic charge $^{\oplus}q$ ; except where $^{\ominus}p$ is 2 and $^{\oplus}q$ is an even integer, then $p_1$ is and $q_1$ is $^{\oplus}q/2$. Thus, for example, where the anion charge $^{\ominus}p$ is 2 and the cationic charge $^{\oplus}q$ is 3, then the anion charge $^{\ominus}p$ is 2 and the cationic charge $^{\oplus}q$ is 3, then the balanced formula (1) contains 2 equivalents of Z, or $p_1$ is 2, and 3 equivalents of the acid anion, or $q_1$ is 3. Of course, where both the cation, Z, and the acid anion are both divalent, i. e. $^{\ominus}p$ and $^{\oplus}q$ are each 2, then there is one mole equivalent of acid anion per mole equivalent of cation, or $p_1$ is 1, and $q_1$ is 2/2 or 1.

Examples of highly advantageous amine cations include

(See Figure Page 6)

$H_3\overset{\oplus}{N}CH_2CH_2OH$,

$H_2\overset{\oplus}{N}(CH_2CH_2OH)_2$,

$H\overset{\oplus}{N}(CH_2CH_2OH)_3$,

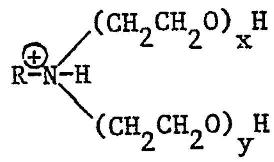

where R is coco fatty alkyl and x + y is about 15,

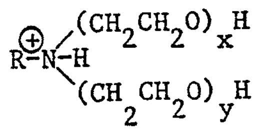

where R is stearyl and x + y is about 15,

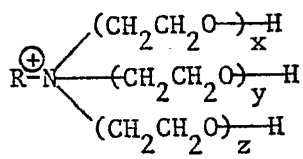

where R is tallow and x + y + z is about 10,

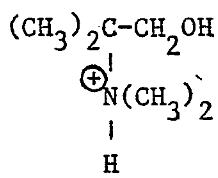

$\overset{\oplus}{N}(CH_3)_4$ ,

$\overset{\oplus}{N}(CH_2CH_2OH)_4$ .

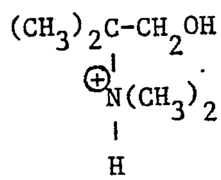

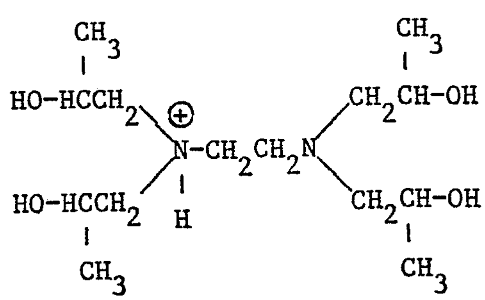

$$H_3\overset{\oplus}{N}CH_2CHOHCHOHCHOHCHOHCH_2OH$$

$$CH_3\overset{\oplus}{N}H_2CH_2CHOHCHOHCHOHCHOHCH_2OH$$

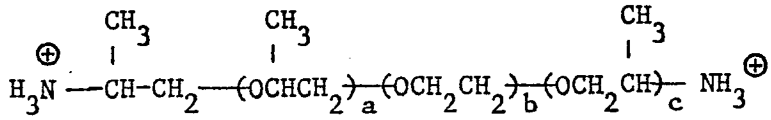

where a + c is 2 to 30 and b is 5 to 40,

$$H_3\overset{\oplus}{N}-CH_2CH_2CH_2\overset{\oplus}{N}H_3$$

and

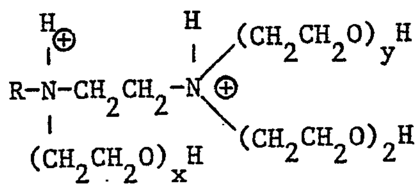

where R is coco fatty alkyl and x + y +z is about 10,

$$H\overset{\oplus}{N}(CH_2COOH)_3.$$

and

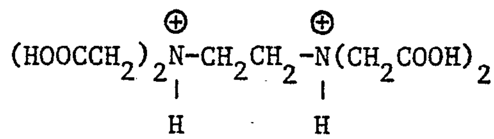

Suitable amines which can be reacted with the gem-perfluoroalkyl containing acids to form useful salts include aminomethane, aminoethane, 1-aminopropane, 2-aminopropane, 1-aminobutane, 1-amino-2-methylpropane, 1.1-dimethylethylamine, 1-aminopentane, isoamylamine, tert.-amylamine, allylamine, dimethylamine, diethylamine, diisopropylamine, trimethylamine, triethylamine, tri-n-butylamine, ethylenediamine, 1.2-propanediamine, trimethylenediamine, 1.3-diaminobutane, 1.4-diaminobutane, hexamethylene diamine, diethylenetriamine, triethylenetriamine, tetraethylene pentamine, polyethyleneimine having an average of about 20, 80, 120 2,000 units, diethylaminopropylamine, ethanolamine, diethanolamine, triethanolamine, ethylenediamine tetraacetic acid, nitrilotrisacetic acid, N-(hydroxyethyl)ethylenediamine, N.N''-bis-(hydroxyethyl)diethylenetriamine, N.N.N'.N'-tetrakis-(2-hydroxypropyl)ethylenediamine N-(2-hydroxypropyl)-ethylenediamine, cyclohexylamine, dicyclohexylamine, 1,8-diamino-p-menthane, and

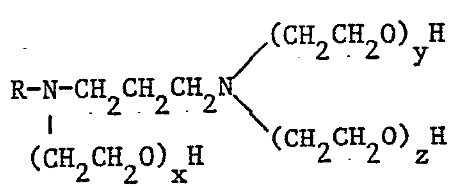

where R is tallow fatty alkyl and x + y + z is 3, 10 or 15.

Suitable perfluoroalkyl acids useful in preparing the instant salts include ;

$$(C_8F_{17}CH_2CH_2S)_2C(CH_3)CH_2CH_2COOH$$

$$(C_6F_{13}CH_2CH_2S)_2C(CH_3)CH_2CH_2COOH$$

$(C_8F_{17}CH_2CH_2S)_2C(CH_3)COOH$

$(C_6F_{13}CH_2CH_2S)_2C(CH_2CH_2COOH)_2$

$[(CF_3)_2CFOCF_2CF_2CH_2CH_2S]_2C(CH_2CH_3)COOH$

$(C_8F_{17}CH_2CH_2OCH_2CH_2CH_2S)_2C(CH_3)CH_2COOH$

$(C_8F_{17}CH_2CH_2N(CH_3)CH_2CH_2S)_2C(CH_3)CH_2COOH$

$(C_8F_{17}CH_2CH_2SO_2)_2C(CH_3)COOH$,

$(C_6F_{13}CH_2CH_2S)_2C-CH_2CH_2COOH$,

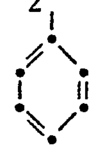

$(C_8F_{17}CH_2CH_2S)_2CHC(CH_3)_2CH_2CH_2COOH$,

and

$(C_8F_{17}CH_2CH_2S)_2CHCOOH$.

The perfluoroalkyl acids useful in preparing the instant salts of formula (1), where B is a covalent bond or alkylene can be prepared in the manner disclosed in US 4,239,915. Those perfluoroalkyl acids useful in preparing the instant salts of formula (1) where B is phenylene, phenoxyalkylene of 7 to 14 carbon atoms, phenylalkenylene of 8 to 14 carbon atoms or phenylene substituted by alkyl of 1 to 4 carbon atoms are prepared in an analogous manner. Thus, two moles of mercaptan, $R_f—R_1—SH$ is reacted per mole of either an aromatic aldehyde or ketone of the formula $R_2—CO—B—COOH$, optionally in the presence of a catalyst, such as hydrogen chloride or zinc chloride, or the corresponding acetal or ketal in the presence of boron trifluoride etherate. The reaction is easily carried out in an inert solvent such as an aliphatic or aromatic hydrocarbon, which may be chlorinated or fluorinated, or in the absence thereof. Suitable solvents include heptane, benzene, methylene chloride, 1.1.2-trifluoro-1.2.2-trichloroethylene, chlorobenzene and the like. Also ethers, such as ethylene glycol dimethyl ether or tetrahydrofuran may be used. The reaction mixture is advantageously kept at a temperature between room temperature and 80 °C under nitrogen until the reaction is completed. If desired, the resulting mercaptal, $(R_f—R_1—S)_2C(R_2)B—COOH$, can be oxidized to the corresponding gem-sulfoxide and further, to the corresponding gem-sulfone by oxidation with the suitable oxidizing agent, such as nitric acid, peracetic acid, potassium monopersulfate, m-chloroperbenzoic acid and the like.

The gem-perfluoroalkyl group containing carboxylic acid amine salts according to formula (1) are formulated as aqueous emulsion concentrates containing 5 % to 40 % by weight of the amine salt.

The aqueous emulsion concentrate is diluted to an application strength such that the emulsion contains 0.01 % to 2 % by weight, more preferably between 0.05 % and 0.30 % by weight og the amine salt, based on the weight of cellulosic or natural or synthetic polyamide substrate.

Suitable cellulosic and natural polyamide substrates for topical application include paper, nonwoven fabrics, paperboard, wood, wood fiber products such as plywood, hair, including wool, hides, leather, and feathers. Synthetic polyamide substrates include nylon fibers and textiles.

For topical application suitable aqueous emulsions contain, advantageously, 0.01 % to 5 %, preferably 0.02 % to 2 %, by weight of the amine salts at use dilution based on the weight of aqueous emulsion. Conventional adjuvants such as water repellent assistants, bacteriostats, coloring agents, surface conditioners and the like, may be included, e. g. in an amount of between about 0.01 % and 5 % by weight in the emulsion. Also, sizing agents, where the emulsion is to be used on cellulosic substrates, may be present in amounts of from 0.01 % to 10 % by weight.

The sizing may be a natural sizing agent such as animal glue, asphalt emulsions, wax emulsions, rosind, starches ; a semisynthetic sizing agent such as a fatty acid salt or complex, a fortified rosin., e. g., tri sodium maleopimaric acid salt, sodium alginate or sodium carboxymethylcellulose ; or a synthetic sizing agent such as an alkylketene dimer, alkylsuccinic anhydrides, polyvinyl alcohol, styrene-maleic anhydride polymers, and the like. Also mixtures therof may be used.

Asphalt emulsions include those obtained from natural deposits of from the residue of crude petroleum distillation and emulsified in an aqueous solution with an emulsifier such as sodium rosinate or fatty amine. Wax emulsions include those prepared from paraffin waxes, optionally blended with rosin size and emulsified with a suitable emulsifier, such as guar gum, gum arabic, stearic acid salts, lignosulfonate salts, alkylamine salts, and the like. Starches include corn starch, potato starch, wheat starch, ethylated corn starch, cationic corn starch corn starch acetate, and the like. Fatty acid salts and complexes include, for example, stearic acid salts, e. g., of aluminium and zirconium, and the corresponding myristic acid, lauric acid, palmitic acid, margaric acid and behenic acid salts and the corresponding chromium complexes of these acids with chromium salts, including the Werner type complexes of a fatty acid, such as stearic or myristic acid with chromium chloride and isopropanol.

Alkylketene dimers include those wherein the alkyl group is between 6 to 23 carbon atoms such as the palmitic, stearic, oleic and myristic ketene dimers, as well as those where the alkyl group is unsaturated or branched chain, and mixtures thereof.

Alkylsuccinic anhydrides include those where the alkyl group is straight or branched chain and may be saturated or unsaturated having between about 6 to 23 carbon atoms, such as the n-hexadecenylsuccinic anhydride, the dodecylsuccinic anhydride, the dodecenylsuccinic anhydride, the isooctadecenylsuccinic anhydride, and the like.

Also alkylcarbamoyl chlorides, such as dilattowamine carbamoyl chloride ; gelatins, cationic aqueous polyurethane emulsions, acrylic resins, stearyl amine surfactants, as known in the art. are suitable sizing agents.

Fillers include materials such as kaolin clay, calcium carbonate, alum, alum, magnesium sulfate, sodium chloride, and the like.

Bacteriostats and fungicides are those commonly used in the paper, leather, fur and textile industries and include halogenated phenols, halogenated carbanilides, o-phenylphenol, salicyclic acid anilide, 2.2′ = methylene-bis(4-chlorophenol), tetraaliphatic ammonium bromides or chlorides, hydroxyquinoline and the like.

Coloring agents include titanium dioxide, and other conventional inorganic pigments, organic pigments, dyes and optical brighteners.

Surface conditioner adjuvants include paper sizing lubricants, such as a fatty acid/polyethylene glycol stearate mixture ; swelling agents, such as an amine oxide swellung agent ; extenders, such as urea ; filler retention aids, such as colloidal silica and methyl cellulose ; and the like.

Also, as discussed below, an emulsifier may also be optionally present in an amount of between about 0.001 % to 3 % by weight in the emulsion.

Thus, suitable aqueous emulsions for topical application contain

a) 0.01 to 5 % by weight of the amine salt ;
b) 0 to 3 % by weight emulsifier ;
c) 0 to 5 % water repellent, filler, bacteriostat, coloring agent or surface conditioner adjuvant ;
d) 0 to 10 % sizing agent, and
e) the remainder water.

These emulsions are applied to the surface of the cellulosic or natural or synthetic polyamide material by conventional techniques, including padding, spraying, coating, washing, and brushing. After application, the treated surface is dried, with or without an intermediate washing stage. The resulting surface is thus rendered water and oil resistant.

For use as an internal sizing agent to obtain oil and water repellency, the dilution of the instant aqueous emulsions advantageously contain from about 0.000 5 to 0.2 or, more preferably, 0.000 5 to 0.1 % by weight of the instant amine salts. The emulsions for dilution may be prepared as a concentrate containing between 5 % and 40 % by weight, preferably 15 to 25 % by weight, of the amine salt, based on the amount of water.

In order to insure emulsion stability of the amine salt in the aqueous medium, the emulsion is advantageously prepared in the presence of a conventional emulsifier. Suitable emulsifiers include cationic, anionic, amphoteric and non-ionic emulsifiers. It is preferred to use non-ionic emulsifiers, such as block copolymers of ethylene oxide and propylene oxide.

The amount of emulsifier used, will, of course depend upon the emulsification characteristics of the amine salt chosen, as well as the desired concentration of the amine salt in the aqueous medium. Where concentrated emulsions are desired, having between 10 and 40 % by weight of the amine salt, it is convenient to use up to 5 % of emulsifier. In many cases, little or no emulsifier need be added to obtain a stable, aqueous emulsion.

Preferably, when present, the emulsifier is present in the emulsion concentrate in an amount between 0.01 and about 3 % by weight.

Suitable emulsifiers for use in the present invention include conventional cationic emulsifiers which are compatible with the active amine salts of the di-perfluoroalkyl group acids, and conventional nonionic emulsifiers.

Preferred emulsifiers are non-ionic emulsifiers including ethoxylated long chain aliphatic amines, ethoxylated long chain aliphatic esters, ethers and thioethers, ethoxylated alkylphenols, block copolymers of ethylene oxide and propylene oxide, and block copolymers of ethylene oxide and propylene oxide on an alkylene polyamine.

Most preferred nonionic emulsifiers are ethylene oxide/propylene oxide block copolymers, polyethoxylated octyl and nonyl phenols, and block copolymers of ethylene oxide and propylene oxide on ethylene diamine. Suitable nonionic emulsifiers are e. g. the condensation products of ethylene oxide with a hydrophobic base formed by the condensation of propylene oxide with propylene glycol which may be represented empirically by the formula

$$HO-(C_2H_4O)_a(C_3H_6O)_b(C_2H_4O)_c-H.$$

Preferred are those condensation products containing about 80 % ethylene oxide units $(-C_2H_4O-)_{a+c}$ and the molecular weight of the polypropylene oxide block $(-C_3H_6HO-)_b$ being about 1.750. (Pluronic F-68, trademark). Further preferred condensation products contain about 70 % ethylene oxide units $(-C_2H_4O-)_{a+c}$ and the molecular weight of the polypropylene oxide block $(-C_3H_6O-)_b$ is about 2.250 (Pluronic F-87, trademark). Another group of suitable condensation products contains about 80 % ethylene oxide units $(-C_2H_4O-)_{a+c}$ and the molecular weight of the polypropylene oxide block $(-C_3H_6O-)_b$ is about 2.750 (Pluronic F-98, trademark).

Further non-ionic emulsifiers comprise the ethoxylation products of t-octylphenols or nonylphenols. Ethoxylated octylphenol having 3 ethylene oxide units (Triton X-35, trademark), ethoxylated octylphenol having 7 or 8 ethylene oxide units (Triton X-114, trademark), ethoxylated nonylphenol having 71 % combined ethylene oxide units by weight of nonylphenyl (Igepal CO-710, trademark) and ethoxylated nonylphenol having 73 % combined ethylene oxide units by weight of nonylphenol (Igepal CO-730, trademark) are suitable examples of said non-ionic emulsifiers.

Also adducts of an ethylendiamine and propylene and ethylene oxide such as the adduct of the approximate formula

$$[H(CO_2H_4)_{17}(CO_3H_6)_{19}]_2NCH_2CH_2N[C_3H_6O)_{19}(C_2H_4O)_{17}H]_2$$

(Tetronic 904, trademark) are suitable emulsifiers for use in the present invention.

While the instant aqueous emulsions are suitable for rendering a variety of cellulosic and natural and synthetic polyamide materials oil and water repellent, the instant emulsions are particularly advantageous in rendering articles made from paper pulp, such as paper trays, paper plates and analogous paper articles, both oleophobic and hydrophobic.

In order to further increase the efficiency of application of the emulsion to the paper pulp, it is desirable to pre-treat the paper pulp with a cationic agent, such as cationically modified starch, which is adsorbed by the paper pulp and, consequently, tends to increase the amount of fluorochemical transferred from the aqueous emulsion to the cellulose substrate.

Suitable cationic agents, conventionally used to pretreat cellulose materials such as paper pulp, include conventional cationic modified starches (Interbond C, Lok-Size 30, Cato 2, Cato 15 and Cato 17 trademarks) ; cationic modified aminoplast resins (Kymene 557, trademark) ; cationic polymers (Santo-Res 21 or Reten-210, trademarks) ; and cationic blocked polyurethanes (Hypol WB-4000, trademark).

Cationic starches are prepared by reacting the starch with amines or quaternary ammonium compounds. Thus, amino ethers of starch are produced by reacting starch with a dialkylaminoalkyl halide which has the amino group in the beta-position, as disclosed, for example, in US 2,970,140 and CA 699,812 both of which describe useful cationic starches. Further useful cationic starches are described in US 2,876,217 and US 3,346,563.

Jointly with the gem-perfluoroalkyl group containing acid salts of the invention, can be added one or more of wide choice of water proofing sizing agents selected from classes such as alkyl anhydrides (Fibron 68, trademark) ; alkyl ketene dimers, (Aquapel 360 XC or Hercon 40, trademarks) ; polyurethane emulsions (Graphsize C, trademark) ; acrylic resins (Carboset, trademark) ; stearyl amine surfactants, (Ethomeen 18/25, trademark, complexed with a fatty acid, e. g. stearic acid ; Neofat 14, Neofat 47 or Hystrene 9718, trademarks).

The amount of adjuvant and sizing agent used for treating paper pulp is of the range specified for topical application, supra.

Thus, for internal sizing of paper pulp suitable aqueous emulsions contain

a) 0.000 5 to 0.1 % by weight of the amine salts of the formula (1) ;
b) 0.000 01 to 0.05 % by weight emulsifier ;

c) 0 to 5 % by weight filler, bacteriostrat, fungicide, coloring agent or surface conditioner adjuvant ;
d) 0 to 10 % sizing agent ; and
e) the remainder water.

The following examples are intended for illustrative purposes only, and are not intended to restrict the scope of the invention in any way. All parts are by weight unless otherwise specified. In the examples, the following abbreviations have been used :

EM1 for Pluronic F-68 (Trademark) ;
EM2 for Tetronic 504 (Trademark) ;
EM3 for Tetronic 701 (Trademark) ;
EM4 for Triton X-35 (Trademark) ;
EM5 for Triton X-114 (Trademark) ;
EM6 for Pluronic F-87 (Trademark) ;
EM7 for Pluronic F-98 (Trademark) ;
EM8 for Pluronic F-127 (Trademark) ;
EM9 for Triton X-165 (Trademark) ;
EM10 for Mykon NRW-3 (Trademark).

## Examples

The following paragraphs describe the preparation of a number of ammonium and amine salts of gem-bis-perfluoroalkyl acids and their usefulness in imparting oil and water repellency to a variety of substrates.

The preparation of many of these useful fluorinated acids is described in US 4,239,915.

A number of other related acids can also be prepared such as :

4.4-Dimethyl-5.5-bis(1.1.2.2-tetrahydroperfluorodecylthio)-pentanoic acid

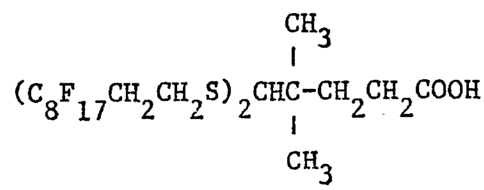

192 g (0.4 mole) of 1.1.2.2-tetrahydroperfluoroalkyldecane-thiol, 28.8 g (0.2 mole) of 4-methyl-4-formylo-pentanoic acid and 100 g of toluene are added to a 500 ml Erlenmeyer flask equipped with a magnetic stirring bar. Anhydrous hydrogen chloride is bubbled through the solution for 2 hours and the reaction mixture is then stirred overnight. The white precipitate which forms is collected and washed with methanol/water (1 : 1), filtered and dried (183 g, 84 % yield, m.p. 83-85 °C).

Analysis for $C_{27}H_{20}F_{34}O_2S_2$ :
calc. C 29.83 %, H 1.84 %, F 59.48 %
found C 29.93 %, H 1.67 %, F 58.91 %

$^1$H-NMR spectrum : 1.08 ppm(s), 1.87 ppm(m), 2.05-3.15(m), 3.52 ppm(s), 10.8(s) in a ratio of approx. 6 : 2 : 10 : 1 : 1.

4-Carboxy-benzaldehyde-bis-(1.1.2.2-tetrahydroperfluorodecylthio)-acetal

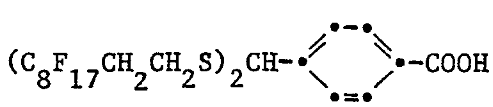

48 g (0.1 mole) of 1.1.2.2-tetrahydroperfluorodecane-thiol, 7.5 g (0.05 mole) of 4-carboxy-benzaldehyde and 120 g of toluene are added to a 500 ml Erlenmeyer flask equipped with a magnetic stirring bar. Anhydrous hydrogen chloride is bubbled through the solution for 2 hours. The white precipitate which results is collected by filtration, washed with methanol/water (1 : 1), filtered again, and dried. (54 g, 99 % yield, m.p. 134°-136 °C).

Analysis for $C_{28}H_{14}F_{34}O_2S_2$ :
calc. C 30.77 %, H 1.28 %, F 59.16 %
found C 30.90 %, H 1.33 %, F 58.33 %

$^1$H-NMR spectrum : 2.85 ppm(m), 5.88 ppm(s), 7.80 (m), 8.14 ppm(m), 10.30 ppm(s) in a ratio of appros. 8 : 1 : 2 : 2 : 1.

In a similar manner, the following compound can be prepared from o-formylphenoxyacetic acid :

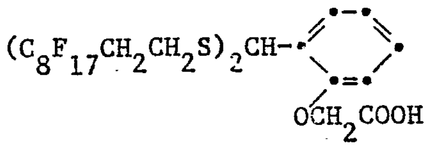

The corresponding sulfoxides and sulfones can be prepared from these gem-bis-perfluoroalkyl-thio acids in the usual way with conventional oxidizing agents such as : hydrogen peroxide, peracetic acid, potassium monopersulfate, m-chloroperbenzoic acid, and the like.

The following examples also describe the preparation of many ammonium and amine salts of a number of these gem-bis-perfluoroalkyl carboxylic acids by conventional means. Additionally, the ammonium and amine salts of this invention can also be prepared by the reaction of ammonium hydroxides or quaternary alkyl ammonium hydroxides with the fluorinated acids. These are shown in examples 1 and 14.

Another alternative method of preparation, is the reaction of the alkali metal salts of the fluorinated acids with quaternary ammonium halides or hydrohalides.

Such as the following :

a) $(R_fCH_2CH_2S)_2CHCOO^{\ominus}Na^{\oplus} + R_3\overset{\oplus}{N}Cl^{\ominus} \rightarrow (R_fCH_2CH_2S)_2CHCOO^{\ominus} \cdot \overset{\oplus}{N}R_3 + NaCl$

b) $(R_fCH_2CH_2S)_2CHCOO^{\ominus}Na^{\oplus} + R_2\overset{\oplus}{N}HCl^{\ominus} \rightarrow (R_fCH_2CH_2S)_2CHCOO^{\ominus}H\overset{\oplus}{N}R_2 + NaCl$

The by-product salts (e. g., NaCl) can be removed by appropriate ion-exchange resins or dialysis membranes.

The examples that follow illustrate a number of the compounds which are included in this invention and their utility. The compounds of this invention are not necessarily limited to those exemplified.

(See Tables, Pages 13 and 14)

Examples

Table I, Gem-perfluoroalkylthio Group Containing Acids

| Designation for subsequent examples | Structure | $R_f$-distribution (%) | | | | |
|---|---|---|---|---|---|---|
| | | $C_6F_{13}$- | $C_8F_{17}$- | $C_{10}F_{21}$- | $C_{12}F_{25}$- | $C_{14}F_{29}$- |
| $A_1$ | $(R_fCH_2CH_2S)_2C(CH_3)CH_2CH_2COOH$ | 6 | 36 | 46 | 11 | 1 |
| $A_2$ | $(R_fCH_2CH_2S)_2C(CH_3)CH_2CH_2COOH$ | 2 | 7 | 71 | 11 | 1 |
| $A_3$ | $(R_fCH_2CH_2S)_2C(CH_3)CH_2CH_2COOH$ | 6 | 30 | 49 | 13 | 2 |
| B | $(R_fCH_2CH_2S)_2CHCOOH$ | 13 | 84 | 3 | - | - |
| C | $(R_fCH_2CH_2S)_2C(COOH)CH_2CH_2COOH$ | 7 | 35 | 46 | 11 | 1 |
| D | $[(CH_3)_2CFOCF_2CF_2CH_2CH_2S]_2C(CH_2CH_3)COOH$ | | | | | |
| E | $[(CH_3)_2CFOCF_2CF_2CH_2CH_2S]_2C(CH_3)CH_2COOH$ | | | | | |
| F | $(C_6F_{13}CH_2CH_2S)_2C_6H_5CH_2CH_2COOH$ | | | | | |
| G | $(C_8F_{17}CH_2CH_2CH_2CH_2S)_2C(COOH)_2$ | | | | | |
| H | $(C_8F_{17}CH_2CH_2OCH_2CH_2CH_2S)_2C(CH_3)CH_2COOH$ | | | | | |
| I | $[C_8F_{17}(CH_2)_2N(CH_3)(CH_2)_3S]C(CH_3)CH_2COOH$ | | | | | |
| J | $(C_8F_{17}CH_2CH_2S)_2C(CH_3)COOH$ | | | | | |
| K | $(C_6F_{13}CH_2CH_2S)_2C(CH_2CH_2COOH)_2$ | | | | | |
| L | $(C_8F_{17}CH_2CH_2SO_2)_2C(CH_3)CH_2CH_2COOH$ | | | | | |
| M | $(C_8F_{17}CH_2CH_2S)_2CHC(CH_3)_2CH_2CH_2COOH$ | | | | | |

Examples continued

Table I, Gem-perfluoroalkylthio Group Containing Acids

| Designation for subsequent examples | Structure | $R_f$-distribution (%) | | | | |
|---|---|---|---|---|---|---|
| | | $C_6F_{13}-$ | $C_8F_{17}-$ | $C_{10}F_{21}-$ | $C_{12}F_{25}-$ | $C_{14}F_{29}-$ |
| N | $(C_8F_{17}CH_2CH_2S)_2CH-$ 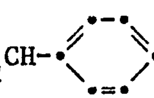 $-COOH$ | | | | | |
| O | $(C_8F_{17}CH_2CH_2S)_2-CH-$ 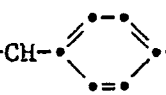 $-CH=CHCOOH$ | | | | | |
| P | $(C_8F_{17}CH_2CH_2S)_2CHCOOH$ | | | | | |

Examples 1-15

This group of examples illustrates a number of the amines and ammonium compounds which are useful for the preparation of the salts of this invention.

Ten gram portions of the fluorochemical acid $A_1$ in table I are weighed into 2 ounce glass jars with the amines listed in Table II, and 30.0 g of methylene chloride. Several stainless-steel balls are added to aid mixing.

The jars are capped, shaken, and placed on a rolling device overnight. The products are then collected by filtration and dried under vacuum at room temperature.

Examples 16-29

These examples demonstrate how the above salts can be converted into useful emulsions or dispersions.

The $R_f$-acid amine salts listed in the above examples (see table II) are each placed in 2 ounce jars with distilled water together with EM1. These mixtures are then stirred at 60-65 °C for 20 minutes. Several stainless-steels balls are added and the jars are placed on a rolling device overnight. These examples of aqueous dispersions are listed in Table III.

(See Tables, Pages 16 to 21)

Table II, Examples 1-15

| Example | Amine | grams of amine used | approx. mole ratio $R_f$-acid:amine | wgt.of dry salt collected | yield % | melting range of salt (°C) | % Fluori calc. |
|---|---|---|---|---|---|---|---|
| 1 | 29.4 % $NH_4OH$ in water | 1.5 | 1:1 | 10.3 g | 97.7 | 106-108 | 61.0 |
| 2 | $H_2NCH_2CH_2OH$ | 0.56 | 1:1 | 10.5 g | 99.0 | 110-130 | 59.7 |
| 3 | $HN(CH_2CH_2OH)_2$ | 1.0 | 1:1 | 9.9 g | 89.6 | 104-131 | 57.7 |
| 4 | $N(CH_2CH_2OH)_3$ | 1.37 | 1:1 | 10.7 g | 93.7 | 104-134 | 55.8 |
| 5 | 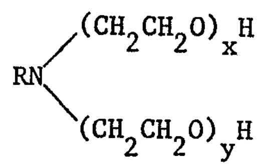 R = coco, x + y≈15 | 8.16 | 1:1 | 11.3 g | 62.3 | 86-90 | 36.1 |
| 6 | 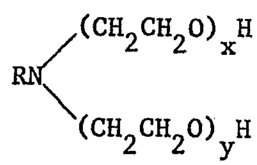 R = stearyl, x + y≈15 | 8.3 | 1:1 | 11.5 g | 62.7 | 93-94 | 35.8 |
| 7 | 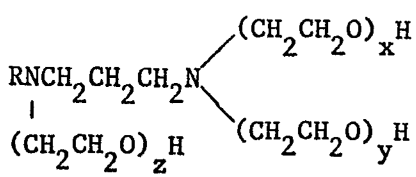 R = tallow, x+y+z≈10 | 1.97 | 1:0.5 | 9.4 g | 78.3 | 99-103 | 53.2 |

Table II, Examples continued

| Example | Amine | grams of amine used | approx. mole ratio $R_f$-acid:amine | wgt. of dry salt collected | yield % | melting range of salt (°C) | % Fluori calc. |
|---|---|---|---|---|---|---|---|
| 8 | $H_2NCHCH_2(OCH_2CH)_a(OCH_2CH_2)_b(OCH_2CH)_cNH_2$<br>$CH_3$ $CH_3$ $CH_3$<br>~76 % ethylene oxide, M.W.=980 | 4.5 | 1:0.5 | 8.9 g | 61.1 | 83-85 | 44.6 |
| 9 | $H_2NCHCH_2(OCH_2CH)_a(OCH_2CH_2)_b(OCH_2CH)_cNH_2$<br>$CH_3$ $CH_3$ $CH_3$<br>~88 % ethylene oxide | 10.8 | 1:0.5 | 16.5 g | 79.1 | 90-96 | 31.6 |
| 10 | $(CH_3)_2-C-CH_2OH$ 80 % in water<br>$N(CH_3)_2$ | 1.34 | 1:1 | 9.3 g | 82.1 | 87-91 | 57.2 |
| 11 | $(CH_3)_2-C-CH_2OH$<br>$NH_2$ | 0.41 | 1:0.5 | 10.3 g | 99.3 | 99-115 | 60.6 |

Table II, Examples continued

| Example | Amine | grams of amine used | approx. mole ratio $R_f$-acid:amine | wgt.of dry salt collected | yield % | melting range of salt (°C) | % Fluori calc. |
|---|---|---|---|---|---|---|---|
| 12 | 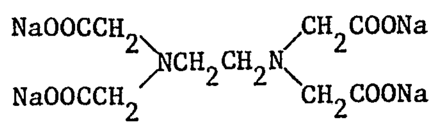 | 3.81 | 1:1 | 13.5 g | 97.8 | >220 | 51.1 |
| 13 | 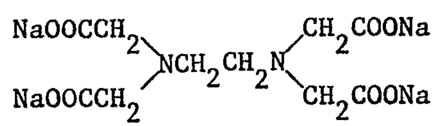 | 1.91 | 1:0.5 | 11.7 g | 98.3 | >220 | 52.7 |
| 14 | $(CH_3)_4NOH$ (20 % in methanol) | 4.17 | 1:1 | 8.9 g | 63.0 | 159-164 | 60.5 |
| 15 | 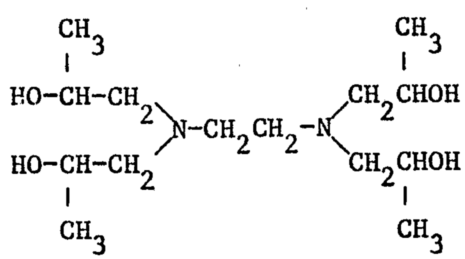 | 1.34 | 1:0.5 | 9.8 g | 86.2 | 89-101 | 56.0 |

Table III, Examples 16-29

Aqueous Dispersions of $(R_fCH_2CH_2S)_2\underset{CH_3}{\underset{|}{C}}-CH_2CH_2COOH$ · Amines Salts

| Example | made from | weight of dist. water | weight of 10 % solution of Pluronic F-68 | calculated solids content | Appearance of dispersion | pH-value of a 1 % dilution (w/v) in water |
|---|---|---|---|---|---|---|
| 16 | 4.0 g example 1 salt | 34.0 g | 3.0 g | 10.5 % | free flowing, opalescent liquid | 8.6 |
| 17 | 4.0 g example 2 salt | 16.5 g | 3.0 g | 18.3 % | free flowing, translucent | 10.0 |
| 18 | 4.0 g example 3 salt | 16.5 g | 3.0 g | 18.3 % | free flowing, translucent liquid | 9.1 |
| 19 | 4.0 g example 4 salt | 22.9 g | 3.0 g | 14.4 % | free flowing, translucent liquid | 7.2 |
| 20 | 4.0 g example 5 salt | 16.0 g | none | 20.0 % | free flowing, milky white liquid | 7.7 |
| 21 | 4.0 g example 6 salt | 16.0 g | 3.0 g | 20.0 % | free flowing, milky white liquid | 9.0 |

Table III, Examples continued

Aqueous Dispersions of $(R_fCH_2CH_2S)_2C—CH_2CH_2COOH \cdot$ Amine Salts
$|$
$CH_3$

| Example | made from | weight of dist. water | weight of 10 % solution of Pluronic F-68 | calculated solids content | Appearance of dispersion | pH-value of a 1 % dilution (w/v) in water |
|---|---|---|---|---|---|---|
| 22 | 4.0 g example 7 salt | 16.3 g | 3.0 g | 18.5 % | pale-yellow cream | 6.5 |
| 23 | 4.0 g example 8 salt | 27.5 g | 0.5 g | 12.7 % | free flowing, opalescent liquid | 8.7 |
| 24 | 4.0 g example 9 salt | 19.5 g | none | 17.0 % | free flowing, opalescent liquid | 9.7 |
| 25 | 4.0 g example 10 salt | 40.0 g | 3.0 g | 9.2 % | fairly viscous opalescent liquid | 9.2 |
| 26 | 4.0 g example 11 salt | 28.2 g | 3.0 g | 12.2 % | opaque viscous liqued | 9.5 |
| 27 | 4.0 g example 12 salt | 28.2 g | 3.0 g | 12.3 % | opalescent viscous liquid | 9.5 |

Table III, Examples continued

Aqueous Dispersions of $(R_fCH_2CH_2S)_2C(CH_3)—CH_2CH_2COOH \cdot$ Amine Salts

| Example | made from | weight of dist. water | weight of 10 % solution of Pluronic F-68 | calculated solids content | Appearance of dispersion | pH-value of a 1 % dilution (w/v) in water |
|---|---|---|---|---|---|---|
| 28 | 4.0 g example 14 salt | 16.5 g | 3.0 g | 18.3 % | fairly viscous opalescent liquid | 9.5 |
| 29 | 4.0 g example 15 salt | 16.4 g | 3.0 g | 18.4 % | fairly viscous opaque liquid | 9.8 |

Example 30

A 1.00 g sample of $(R_fCH_2CH_2S)_2CHCOO^{\ominus\oplus}N(CH_3)_4$ is made into an aqueous dispersion, in the same manner as examples 16-29, with 4.02 g dist. water, 0.10 g diethanolamine, and 0.75 g of a 10 % solution of EM-1.

$R_f$ = 6 % $C_6F_{13}$, 36 % $C_8F_{17}$, 46 % $C_{10}F_{25}$, 1 % $C_{14}F_{29}$.

Example 31

By an alternative method, the salt and dispersion are formed simultaneously, in one operation, by weighting 6.84 g dist. water, 0,17 g of diethanolamine, 1.20 g of 10 % aqueous EM-1, and 1.59 g of acid B (in table I) into a 2 ounce jar. The contents are stirred at 60 °C for 10 minutes. The jar is capped and placed on a rotating device for several days. A viscous, slightly cloudy liquid results. At 1 % in water, the pH-value is 10.0.

Examples 32-37

Several dispersing and/or wetting aids are utilized in these examples which are listed in table IV. Two grams of acid $A_2$ from table I, 0.162 g of diethanolamine, and the amount and type of wetting aid shown in table IV are mixed with 2 grams of destilled water at 55 °C ; then diluted with additional water (see table IV). After additional mixing at 55 °C ; the dispersions, in 2 ounce jars, are rotated overnight.

Example 38

In this example, the salt and dispersion are also prepared in one operation.

To a 20-liter glass reactor, equipped with a motor driven stirrer, thermometer, nitrogen inlet, and vent, are charged 1,755 g of distilled water and 195 g of EM-1 (powder). This is stirred at 35-40 °C until dissolved. Then 273.3 g (2.6 moles) of diethanolamine and 8,900 g of distilled water are added with stiring. Then 3,702 g (2.25 moles) of a paste containing 74 % acid $A_3$ from table I in water and methanol is charged in followed by 174.4 g of distilled water. The mixture is stirred at 60-65 °C for 4 hours. A viscous, light-straw colored, translucent liquid resulted.

Solids : calculated = 21.3 %, found = 21.5 %

Fluorine in solids : calculated = 53.0 %, found = 53.50 %.

The above dispersion formed a clear bluish solution when diluted to 1 % (as is) in distilled water. The pH-value is 9.7.

(See Table Page 23)

Table IV, Examples 32-37

Dispersing/wetting aids for $(R_fCH_2CH_2S)_2CCH_2CH_2COOH$ — diethanolamine salt
$CH_3$ (on C)

| Example | Dispersing/wetting aid | | additional water added | total batch size | Appearance of dispersion |
|---|---|---|---|---|---|
| | amount* | type | | | |
| 32 | mix. { 0.62 g<br>1.38 g | EM 2<br>EM 3 | 6.36 g | 12.25 g | white paste |
| 33 | 1.0 g | EM 4 | 12.64 g | 17.8 g | white paste |
| 34 | 1.0 g | EM 5 | 12.64 g | 17.8 g | somewhat viscous, free-flowing, hazy liquid |
| 35 | 1.0 g | EM 1 | 12.64 g | 17.8 g | somewhat viscous, free-flowing hazy liquid |
| 36 | 1.0 g | EM 6 | 12.64 g | 17.8 g | somewhat viscous, free-flowing, hazy liquid |
| 37 | 1.0 g | EM 8 | 12.64 g | 17.8 g | somewhat viscous, free-flowing, hazy liquid |

Examples 39-42

Aqueous dispersions of acid $A_3$ (listed in table I) are made with several other amines. The preparation of the salts and dispersions is carried out in one operation. These examples are listed in table V. Distilled water, the amine, wetting aid, and fluorinated acid are blended together in a 2 ounce jar, stirred for 10 minutes at 60 °C, and placed on a rotating device overnight.

Example 43

The use of another of the fluorinated acids is described in this example.

By the method described in Example 31, a dispersion is prepared from 7.92 g of distilled water, 0.34 g of diethanolamine, 2.0 g of a 10 % aqueous. EM 7 solution, and 2.0 g of acid C in table 1. An opalescent liquid results.

Examples 44-51

The use of a number of other fluorinated acids and amines is described in this group of examples.

Using the methods and techniques described in examples 1-29, the examples listed in Table VI are prepared.

(See Tables, Pages 25 and 26)

Table V, Examples 39-42

| Example | Wgt. of $R_f$-acid | Wgt. of water | Wgt. of EM 1 | Amine |  | Dispersion |  | pH-value of a 1 % dilution in water |
|---|---|---|---|---|---|---|---|---|
|  |  |  |  | amount | type | calc. % solids | appearance |  |
| 39 | 4.0 g | 15.96g | 3.0 g | 0.54 g | triethanolamine | 20.6 | free-flowing, milky white liquid | 10.0 |
| 40 | 4.0 g | 16.18g | 3.0 g | 0.32 g | 2-amino-2-methyl-1-propanol | 19,7 | slightly viscous, opalescent liquid | 9.5 |
| 41 | 4.0 g | 15.50g | 3.0 g | 1.00 g | N-coco-1,3-propane diamine | 22.6 | viscous, creamy, white liquid | 10.2 |
| 42 | 4.0 g | 15.22g | 3.0 g | 1.28 g | bis-(2-hydroxyethyl)-tallowamine | 23.7 | separated | - |

Table VI, Examples 44-51

| Example | $R_f$-acid as listed in Table I | Amine | Product |
|---|---|---|---|
| 44 | D | stearyl amine | $[(CF_3)_2CFOCF_2CF_2CH_2CH_2S]_2C(CH_2CH_3)COO^{\ominus}\cdot N_3N^{\oplus}-C_{18}H_{37}$ |
| 45 | E | diethylamine | $[(CF_3)_2CFOCF_2CF_2CH_2CH_2S]_2C(CH_3)CH_2COO^{\ominus}\cdot H_2N^{\oplus}(CH_2CH_3)_2$ |
| 46 | F | guanidine | $[(C_6F_{13}CH_2CH_2S)_2C(phenyl)CH_2CH_2COO^{\ominus}]_2\cdot(H_3N^{\oplus})_2C{=}NH$ |
| 47 | G | triethylamine | $(C_8F_{17}CH_2CH_2CH_2CH_2S)_2C(COO^{\ominus})_2\cdot[HN^{\oplus}(CH_2CH_3)_3]_2$ |
| 48 | H | pyridine | $(C_8F_{17}CH_2CH_2OCH_2CH_2CH_2S)_2C(CH_3)CH_2COO^{\ominus}\cdot$ 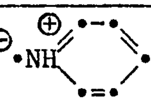 |
| 49 | I | melamine | $\{[C_8F_{17}(CH_2)_2N(CH_3)(CH_2)_3S]C(CH_3)CH_2COO^{\ominus}\}_3\cdot H_3N^{\oplus}-$ 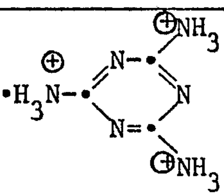 |
| 50 | J | poly(ethylenimine) | $[(C_8F_{17}CH_2CH_2S)_2C(CH_{33})COO^{\ominus}]_{180}\,{}^{\oplus}NH_3-(CH_2CH_2N^{\oplus}H_2)_{179}H$ |
| 51 | K | N-coco-propylenediamine | $(C_6F_{13}CH_2CH_2S)_2C(CH_2CH_2COO^{\oplus})_2\cdot[H_2NCH_2CH_2CH_2N^{\oplus}H_2-R]_2$ where R = coco |

Example 52

This example describes the use of a bis-sulfone type of acid.

Using the method described in example 31, a dispersion is prepared from 6.64 g of distilled water, 2.20 g of 10 % aqueous EM 1 0.21 g of diethanolamine, and 2.00 g of acid L (from table I). A fuid translucent white liquid results (at 22.0 % solids). A 1 % dilution in water produced a hazy solution at a pH-value of 9.7.

The following examples illustrate how the compounds and dispersions of the previous examples can be used to impart oil and water repellent properties to a variety of substrates.

## Application Examples

Example 53

Samples of fluorochemical dispersion of the type described in example 38 are added to a 10 % aqueous solution of paper maker's starch (pH-value adjusted to 9.6 with 10 % aqueous NaOH). This starch solution is applied to unsized paper by padding (paper dipped through starch solution, then passed through single nip rollers). The resulting sheets of paper are dried at ambient conditions for 15 minutes, then for 3 minutes at 93 °C in an « Emerson Speed Drier » (heated metal plate with canvas cover). Table VII lists the results.

Table VII, Example 53

| Sample | amount of fluorochem. dispersion added 1) | dry weight pick-up of starch | amount of fluorochem. solids to wgt. of paper | oil kit number 2), 3) | Gurley-Cobb test-water absorbed 2),4) |
|---|---|---|---|---|---|
| a | none | 5.8 % | none | 0 | 121. $g/M^2$ |
| b | 6 ml | 5.2 % | 0.036 % | 5 | 12.8 $g/M^2$ |
| c | 8 ml | 4.2 % | 0.039 % | 6 | 13.7 $g/M^2$ |
| d | 10 ml | 5.1 % | 0.060 % | 6 | 12.6 $g/M^2$ |

Notes :

1) Amount shown is pre-diluted 1 : 1 with distilled water and added into 100 ml starch solution at 66 °C.
2) Measured on side facing canvas in drier.
3) OIL KIT for Surface Oil Resistance Tests (TAPPI method UM-557).

An easily made kit of twelve solutions of varying proportions of Castor Oil, Toluene, and Heptane is useful in comparing surface oil resistance.

| Kit No. | Volume Castor Oil | Volume Toluene | Volume Heptane |
|---|---|---|---|
| 1 | 200 | 0 | 0 |
| 2 | 180 | 10 | 10 |
| 3 | 160 | 20 | 20 |
| 4 | 140 | 30 | 30 |
| 5 | 120 | 40 | 40 |
| 6 | 100 | 50 | 50 |
| 7 | 80 | 60 | 60 |
| 8 | 60 | 70 | 70 |
| 9 | 40 | 80 | 80 |
| 10 | 20 | 90 | 90 |
| 11 | 0 | 100 | 100 |
| 12 | 0 | 90 | 110 |

The « kit value » is defined as the highest number solution that will stand on the surface of the plate for 15 seconds in the form of drops without failing. Failure is detected by pronounced darkening caused by penetration. The darkening of even a small fraction of the area under the drop is considered failure.

4) Cobb size test, ASTM method D-3285-73 ; measurements made after 15 second exposure to water.

Example 54

A 4 % solution of cationic starch is distilled water is prepared by cooking in a double boiler. When cooled, 50 ml of this starch solution is placed into a beaker followed by 50 ml of a 2 % as is (0.426 % solids) solution of fluorochemical dispersion of the type described in example 38. This mixture is then pad applied (as is example 53) to unsized, bleached, kraft paper at 100 % wet pick-up. The resulting paper is dried (as in example 53) at 94 °C for 20 minutes. The treated paper demonstrated an oil kit number (TAPPI UM-557) of 12 and a Gurley-Cobb sizing value of 24 gm/$M^2$ of water absorbed, compared to a kit number of 0 and Cobb test of 813 g/$M^2$ with cationic starch alone with no fluorochemical.

Example 55

This example demonstrates the utility of these dispersions as internal sizes.

Six grams of dry bleached Kraft pulp are diluted in 289 ml distilled water and thoroughly dispersed in a blender. To this pulp slurry is added 3.6 ml of a 1 % dilution (as is) of the dispersion from example 31 in distilled water. This is mixed in for 5 minutes, then 6 ml of a 1 % aqueous solution of cooked cationic starch is added. This is mixed together for an additional 5 minutes. To this, 1.2 ml of a 1.5 % (on solids) dilution of an alkyl ketene dimer emulsion is added as a water repellent adjuvant. This is mixed in for another 10 minutes. The resulting slurry is diluted with an additional 500 ml of distilled water and mixed again. This mixture is then poured over a 100 mesh wire screen, with a vacuum applied from below which pulled the water from the pulp mixture to form a sheet on the screen. The wet sheet is removed from the screen and dried between another screen and hard surface at a pressure of approximately 0.4 lbs/in.$^2$ at 110 °C for 1 1/2 hours. The paper formed in this manner, shows a TAPPI method UM-557 oil kit number of 4 (see example 53). One ml of hot (110 °C) corn oil placed on the paper remained on the surface and does not penetrate for 12 minutes. Similarly, 1 ml of hot (80 °C) water containing 0.5 % of EM 9 placed on the paper does not penetrate for 20 minutes. Whereas paper made in the same manner, including the cationic starch and water repellent adjuvant but without the fluorochemical dispersion, demonstrats an oil kit number of $< 1$, and holds the hot corn oil and hot water/EM 9 solution for less than one minute (begins to penetrate as soon as applied).

Examples 56-58

Three pad bath solutions are made up, each containing : 8.6 ml of the same kind of fluorochemical dispersion as described in example 38, 2.5 ml of a melamine-formadehyde resin (at 80 % actives), 0.4 ml of a hydroxy-alkylamine-hydrochlorid accelerator, 0.1 ml of EM 10, and 88.4 ml of distilled water.

These pad baths are then applied to the substrates shown in table VIII at a 50 % wet pickup (one pass through bath into single nip rollers). The treated samples are dried at 110 °C for 2 minutes and cured at 163 °C for 3 minutes. Water and oil repellency measurements are then made. The results are shown in Table VIII. Application of the same system without the fluorochemical dispersion results in no oil water repellency (zero ratings).

(See Table, Page 29)

Table VIII, Examples 56-58

| Example | Substrate | Oil Repellency Rating 1) | Water Repellency Rating 2) |
|---|---|---|---|
| 56 | fiber reinforced cellulose based non-woven fabric | 5 | 50 |
| 57 | 100 % filament polyamid poncho | 4 | 90 |
| 58 | 100 % spun dimethyl therephthalat/ethylene-glycol condensat | 4 | 80 |

1) Oil Repellency : AATCC test method no. 118-1975
2) Water Repellency : AATCC test method no. 22-1974

Examples 59-63

For purposes of comparison, mono-perfluoroalkyl chain containing carboxylic acid compounds and a sample of a commercial fluorochemical paper size (twin-$R_f$-tailed phosphate ester, ammonium salt) are tested against the gem-bisperfluoroalkylthio group containing acids of this invention (Examples 59-62). The compounds are applied as aqueous dispersions or solutions and evaluated in the manner similar to that described in example 55 (i. e. internal size). These tests are shown in table IX.

(See Table Page 30)

Table IX, Examples 59-63

| Example | Fluorochemical | fluorine on wgt. of paper | cationic starch in paper | alkyl ketene dimer adjuvant in paper | oil Kit number | Hold-out tests observed for up to 20 minutes | |
|---|---|---|---|---|---|---|---|
| | | | | | | hot, 110°C corn oil | hot, 80°C water + 0.5 % EM 9 |
| 59 | diethanolamine salt of $(C_8F_{17}CH_2CH_2S)_2C(CH_3)CH_2-CH_2)COOH$ aqueous dispersion prepared as in example 31 | 0.08 % | 1,0 % | 0.3 % | 5 | >20 min. | 20 min. |
| 60 | diethanolamine salt of $C_8F_{17}CH_2CH_2SCH_2CH_2COOH$, aqueous solution | 0.08 % | 1.0 % | 0.3 % | 1-2 | <1 min. | <1 min. |
| 61 | diethanolamine salt of $C_8F_{17}CH_2CH_2SCH_2COOH$, aqueous solution | 0.08 % | 1.0 % | 0.3 % | 2 | 2 min. | 3 min. |
| 62 | aqueous dispersion of the same type as in example 38 (diethanolamine salt of acid $A_3$ in table I) | 0.04 %<br>0.04 %<br>0.06 %<br>0.06 % | 1.0 %<br>1.0 %<br>1.0 %<br>1.0 % | 0.3 %<br>0.45 %<br>0.3 %<br>0.45 % | 4<br>3-4<br>5<br>5 | 20 min.<br>>20 min.<br>>20 min.<br>>20 min. | 20 min.<br>15-20 min.<br>20 min.<br>20 min. |
| 63 | bis-perfluoroalkyl-phosphate ester, ammonium salt | 0.04 %<br>0.04 %<br>0.06 % | 1.0 %<br>1.0 %<br>1.0 %<br>1.0 % | 0.3 %<br>0.45 %<br>0.3 %<br>0.45 % | 2-3<br>3<br>5<br>4 | <1 min.<br><1 min.<br>20 min.<br>20 min. | 15-20 min.<br>15-20 min.<br>20 min.<br>>20 min. |

Example 64

In this example, the salt of the sulfone type acid (L) is evaluated as an internal paper size.

The fluorochemical dispersion of example 52 is applied and tested by the method described in example 55. At an application level of 0.78 % dispersion to weight of dry pulp, and oil kit value of 5 is obtained, the sheet holds out the hot corn oil for more than 20 minutes, and holds out the hot water EM 9 solution for about 20 minutes.

Example 65

In this example, the salt of another acid (M) is also evaluated as an internal size.

By the method described in example 31, a dispersion is prepared from 14.05 g of distilled water, 3.00 g of 10 % aqueous EM 1, 0.43 g of diethanolamine, and 4.00 g of acid M (from table I). This produces a translucent white paste (22 % solids). At 1 % in water, the pH-value is 9.5.

This fluorochemical dispersion is then evaluated by the method of example 55. It shoes the same performance as listed in example 64.

Example 66

By the method described in example 54, the dispersion from example 65 (acid M) is evaluated as an external paper size. A mixture of 50 ml of 4 % cationic starch, 20 ml of the fluorochemical dispersion (diluted to 5 % as is), and 30 ml of distilled water is pad applied to the paper. The dried sheet shows an oil Kit number of 9, and a Gurley-Cobb sizing value of 57 g/$M^2$ (water absorbed).

Example 67

In this example, the dispersion of acid N (from table I) is prepared and evaluated as an external paper size.

By the method described in example 31, an aqueous fluorochemical dispersion is prepared from 14.03 g of distilled water, 3.00 g of a 10 % solution of EM 1, 0.42 g of diethanolamine, and 4.00 g of acid N (aromatic type). The resulting product of a milky white (almost translucent paste) giving a pH-value of 9.28 at 1 % in water.

This is applied and tested as above (example 66). The dried sheet of paper has an oil Kit number of 6 and a Gurley-Cobb sizing value of 80 g/$M^2$.

Example 68

In this example, an aqueous dispersion is prepared from acid P (in table I) and also evaluated as an external size on paper.

By the method described in example 31, 14.16 g of distilled water, 3.00 g of a 10 % solution of EM 1, 0.46 g of diethanolamine, and 4.00 g of acid P (with 61.87 % F found) are combined to form a translucent light-straw colored liquid which has a pH-value of 9.07 when diluted to 1 % in water.

When applied and tested as in example 66, the resulting treated paper has an oil Kit value of 3 and Cobb-size rating of 103 g/$M^2$ (water absorbed).

Example 69

In this example the acid of the formula

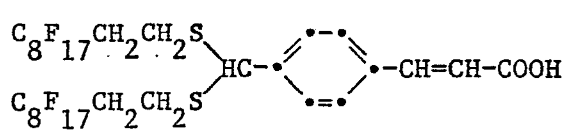

is prepared by the following procedure :

15.0 g (0.085 moles) of p-Formylcinnamic acid, 82.5 g (0.17 moles) of n-$C_8F_{17}CH_2CH_2SH$ and 200.0 g of acetic acid are charged to the reaction flask and the temperature is raised and held at 81 °C to dissolve the p-formylcinnamic acid. Hydrogen chloride gas is introduced for 10 minutes during which time a white solid precipitated out of solution. The resulting solids are filtered, washed with aqueous methanol and dried in a 60 °C vacuum oven overnight. The yield is 95.9 % white powder.

| Elemental analysis : | % calculated | % found |
|---|---|---|
| | C 32.21 | 32.34 |
| | H 1.44 | 1.32 |
| | F 57.75 | 56.83 |
| | S 5.73 | 6.55 |

Example 70

As in Example 68, an aqueous emulsion is prepared from 13.77 g of distilled water, 4.40 g of a 10 % solution of EM 1, 0.56 g of diethanolamine, and 4.00 g of acid « O », listed in table I, and prepared in accordance with Example 69. This produces a white liquid which has a pH-value of 9.3 at 1 % water.

When applied and tested as above, the treated paper has an oil kit number of 8 und a Cobb-size value of 17 g/M$^2$ (water absorbed).

**Claims**

1. A compound of the formula

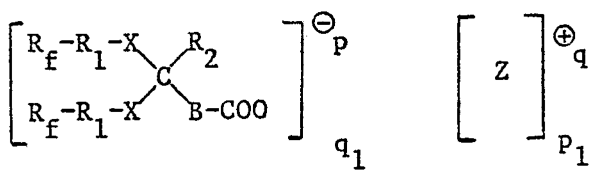

wherein

$R_f$ is perfluoroalkyl of 4 to 18 carbon atoms or perfluoroalkoxy-perfluoroalkylene of 4 to 18 carbon atoms ;

$R_1$ is alkylene of 1 to 12 carbon atoms, alkylenethioalkylene of 2 to 12 carbon atoms, alkyleneoxyalkylene of 2 to 12 carbon atoms, or alkyleneiminoalkylene of 2 to 12 carbon atoms where the imino nitrogen atom contains as a third substituent, hydrogen or alkyl of 1 to 6 carbon atoms ;

X is —S—, —SO— or —$SO_2$— ;

$R_2$ is hydrogen, alkyl of 1 to 6 carbon atoms, phenyl, alkyl substituted phenyl of up to 18 carbon atoms, or is the carboxylic acid group —B—COOH, or

the carboxylate anion —B—$COO^{\ominus}$ ;

B is a covalent bond, or alkylene of up to 18 carbon atoms, phenylene, phenoxyalkylene of 7 to 14 carbon atoms, phenylalkylene of 8 to 14 carbon atoms, or phenylene substituted by alkyl of 1 to 4 carbon atoms ;

Z is the ammonium or amine cation ;

$^{\ominus}p$ is the anionic charge of the gem-perfluoroalkyl group containing acid and is 1 or 2,

$^{\oplus}q$ is the cationic charge of the ammonium or amine group Z and has a value of 1 to 200 ; and

the cationic mole equivalent, $p_1$ is equal to $^{\ominus}p$ and the anion mole equivalent, $q_1$, is equal to $^{\oplus}q$, with the proviso that where $^{\ominus}p$ is 2 and $^{\oplus}q$ is an even integer, then $p_1$ is 1 and $q_1$ is $^{\oplus}q/2$.

2. A compound according to claim 1, wherein $R_f$ is perfluoroalkyl of 6 to 14 carbon atoms, $R_1$ is alkylene of 2 to 8 carbon atoms, X is —S— or —$SO_2$—, $R_2$ is hydrogen or alkyl of 1 to 4 carbon atoms, —B— is a covalent bond or alkylene of 1 to 6 carbon atoms, $^{\ominus}p$ is 1 and $^{\oplus}q$ is 1 to 4.

3. A compound according to claim 1, wherein $R_f$ is perfluoroalkyl of 6 to 12 carbon atoms, $R_1$ is alkylene of 2 to 4 carbon atoms, X is —S— or —$SO_2$—, $R_2$ is hydrogen or alkyl of 1 or 2 carbon atoms, —B— is a covalent bond or alkylene of 1 to 3 carbon atoms, $^{\ominus}p$ is 1 and $^{\oplus}q$ is 1 or 2.

4. A compound according to claim 1, wherein Z is the cation of ammonia or a water-soluble mono- or polyamine.

5. A compound according to claim 4, wherein Z is

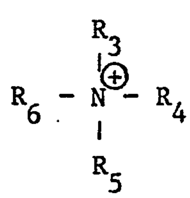

wherein $R_3$ is hydrogen, alkyl of 1 to 5 carbon atoms, alkyl of 2 to 6 carbon atoms substituted by 2 to 6 hydroxy groups, alkenyl of 3 to 5 carbon atoms, cycloalkyl of 5 to 7 carbon atoms, —$C_1$—$C_3$—alkylene—COOH,

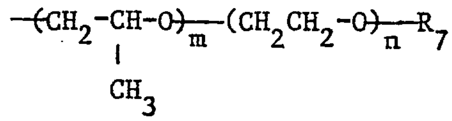

where m is 0 to 6, n is 0 to 30, and $R_7$ is hydrogen, alkyl of 1 to 3 carbon atoms or alkanoyl of 2 to 4 carbon atoms, or is

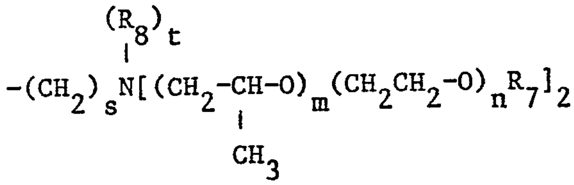

wherein s is 2 to 6, $R_8$ is hydrogen or alkyl of 1 to 3 carbon atoms, t is 0 or 1 and m, n and $R_7$ are as above defined ;

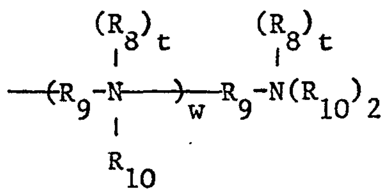

where $R_9$ is alkylene of 2 to 8 carbon atoms, cyclohexylene or di($C_1$ to $C_3$-alkylene)cyclohexylene, w is 0 to 198, $R_{10}$ is hydrogen, alkyl of 1 to 3 carbon atoms, alkyl of 2 to 6 carbon atoms substituted by hydroxy, or

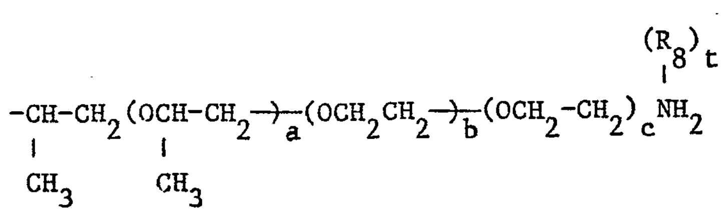

where a is 1 to 20, is 2 to 50, c is 2 to 20 and $R_8$ and t are as defined above ;

$R_4$ and $R_5$ are independently hydrogen, alkyl of 1 to 5 carbon atoms or

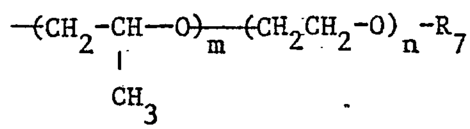

where m, n and $R_7$ are as defined above ; or

$R_4$ and $R_5$ taken together with the nitrogen to which they are attached represent morpholino ; and

$R_6$ is alkyl of 1 to 5 carbon atoms ; hydrogen when at least one of $R_3$, $R_4$ and $R_5$ is other than hydrogen ; alkyl of 6 to 23 carbon atoms when $R_3$ is other than hydrogen or alkyl of 1 to 5 carbon atoms ;

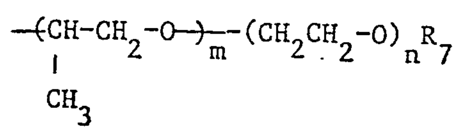

where m, n and $R_7$ are as defined above ;

or $R_4$, $R_5$ and $R_6$, taken together with the nitrogen which they are attached represent pyridyl ; with the proviso that the total value of t equals $^{\oplus}q - 1$.

6. A compound according to claim 5, wherein $R_3$ is $(CH_2CH_2—O)_nH$ where n is 1 to 10, $R_4$ and $R_5$ are

independently hydrogen of $(CH_2CH_2—O)_nH$ where n is 1 to 10 and $R_6$ is hydrogen or $(CH_2CH_2—O)_nH$ where n is 1 to 10.

7. A compound according to claim 5, wherein $R_3$ is of the formula $(CH_2CH_2—O)_nH$ where n is 1 to 10, $R_4$ is $(CH_2CH_2—O)_nH$ where n is 1 to 10, $R_5$ is hydrogen or $(CH_2CH_2—O)_nH$ where n is 1 to 10 and $R_6$ is alkyl of 6 to 20 carbon atoms.

8. A process for preparing the compound according to claim 1 by reacting $q_1$ moles of the acid of the formula

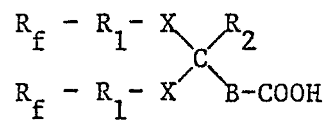

or the alkali or alkaline earth metal salt thereof, with $p_1$ moles of ammonia or amine of the formula Z, in the form of its free base or a salt thereof, in the presence of an inert diluent, at a temperature between 0 °C and 100 °C.

9. An aqueous emulsion concentrate containing 5 % to 40 % by weight of the amine salt according to claim 1.

10. An aqueous emulsion or dispersion for topical application to a cellulosic or natural or synthetic polyamide substrate, to render the substrate oil and water repellent comprising

a) 0.01 to 5 % by weight of the amine salt according to claim 1 ;
b) 0 to 3 % by weight emulsifier ;
c) 0 to 5 % by weight filler, water repellent assistant, bacteriostat, coloring agent or surface conditioner adjuvant ;
d) 0 to 10 % by weight sizing agent, and
e) the remainder water.

11. An aqueous emulsion for the internal sizing of paper pulp, to render the same oil and water repellant, comprising

a) 0.000 5 to 0.1 % by weight of an amine salt according to claim 1 ;
b) 0.000 01 to 0.05 % by weight emusifier ;
c) 0 to 5 % by weight filler, bacteriostat, fungicide, coloring agent or surface conditioner adjuvant ;
d) 0 to 10 % sizing agent ; and
e) the remainder water.

12. A process for rendering a cellulosic or natural or synthetic polyamide substrate oil and water repellent comprising the steps of contacting said substrate with an effective amount of an aqueous emulsion according to claim 10, and drying the treated substrate.

13. A process for the internal sizing of paper pulp, comprising contacting said paper pulp with an effective amount of an aqueous emulsion according to claim 11, to render the pulp oil and water repellent, and subsequently drying the pulp.

14. A water and oil repellent cellulosic, or natural or synthetic polyamide, substrate produced according to the process of claim 12.

15. An oil and water repellent paper pulp article produced according to the process of claim 13.

**Ansprüche**

1. Verbindungen der Formel

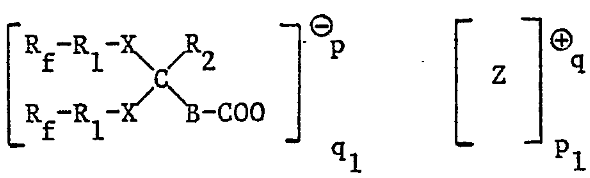

worin

$R_f$ Perfluoroalkyl mit 4 bis 18 Kohlenstoffatomen oder Perfluoroalkoxy-perfluoroalkylen mit 4 bis 18 Kohlenstoffatomen ;

$R_1$ Alkylen mit 1 bis 12 Kohlenstoffatomen, Alkylenthioalkylen mit 2 bis 12 Kohlenstoffatomen, Alkylenoxyalkylen mit 2 bis 12 Kohlenstoffatomen oder Alkyleniminoalkylen mit 2 bis 12 Kohlenstoffatomen, worin das Stickstoffatom der Iminogruppe als dritten Substituenten Wasserstoff oder Alkyl mit 1 bis 6 Kohlenstoffatomen enthält ;

X —S—, —SO— oder —$SO_2$— ;

$R_2$ Wasserstoff, Alkyl mit 1 bis 6 Kohlenstoffatomen, Phenyl, alkylsubstituiertes Phenyl bis zu 18 Kohlenstoffatomen oder die Carbonsäuregruppe —B—COOH oder das Carboxylatanion —B—$COO^{\ominus}$ ;

B eine kovalente Bindung Alkylen bis zu 18 Kohlenstoffatomen, Phenylen, Phenoxyalkylen mit 7 bis 14 Kohlenstoffatomen, Phenylalkylen mit 8 bis 14 Kohlenstoffatomen oder alkylsubstituiertes Phenylen, wobei der Alkylrest 1 bis 4 Kohlenstoffatome enthält ;

Z Ammonium oder ein Aminkation ;

$^{\ominus}p$ die negative Ladung der geminale Perfluoroalkylgruppen enthaltenden Säure und 1 oder 2 ;

$^{\oplus}q$ die positive Ladung des Ammonium- oder Aminrestes Z und 1 bis 200 ;

das kationische Mol-Aequivalent $p_1$ gleich $^{\oplus}p$ und das anionische Mol-Aequivalent $q_1$ gleich $^{\oplus}q$ ist, wobei, wenn $^{\ominus}p$ 2 ist und $^{\oplus}q$ dieselbe Bedeutung hat, $p_1$ 1 und $q_1$ $^{\oplus}q/2$ ist.

2. Verbindungen nach Anspruch 1, worin $R_f$ Perfluoroalkyl mit 6 bis 14 Kohlenstoffatomen, $R_1$ Alkylen mit 2 bis 8 Kohlenstoffatomen, X —S— oder —SO—, $R_2$ Wasserstoff oder Alkyl mit 1 bis 4 Kohlenstoffatomen, —B— eine kovalente Bindung oder Alkylen mit 1 bis 6 Kolenstoffatomen, $^{\ominus}p$ 1 und $^{\oplus}q$ 1 bis 4 ist.

3. Verbindungen nach Anspruch 1, worin $R_f$ Perfluoroalkyl mit 6 bis 12 Kohlenstoffatomen, $R_1$ Alkylen mit 2 bis 4 Kohlenstoffatomen, X —S— oder —$SO_2$—, $R_2$ Wasserstoff oder Alkyl mit 1 oder 2 Kohlenstoffatomen, B eine kovalente Bindung oder Alkylen mit 1 bis 3 Kohlenstoffatomen, $^{\ominus}p$ 1 und $^{\ominus}q$ 1 oder 2 ist.

4. Verbindungen nach Anspruch 1, worin Z Ammonium oder das Kation eines wasserlöslichen Mono- oder Polyamins ist.

5. Verbindungen nach Anspruch 4, worin Z der Formel

$$R_6-\overset{R_3}{\underset{R_5}{N^{\oplus}}}-R_4$$

entspricht, worin $R_3$ Wasserstoff, Alkyl mit 1 bis 5 Kohlenstoffatomen, mit 2 bis 6 Hydroxygruppen substituiertes Alkyl mit 2 bis 6 Kohlenstoffatomen, Alkenyl mit 3 bis 5 Kohlenstoffatomen, Cycloalkyl mit 5 bis 7 Kohlenstoffatomen, —$C_1$—$C_3$—Alkylen—COOH,

$$-(CH_2-\underset{CH_3}{CH}-O)_m-(CH_2CH_2-O)_n-R_7$$

worin m 0 bis 6, n 0 bis 30 und $R_7$ Wasserstoff, Alkyl mit 1 bis 3 Kohlenstoffatomen oder Alkoxy mit 2 bis 4 Kohlenstoffatomen, oder $R_3$

$$-(CH_2)_s\overset{(R_8)_t}{N}[(CH_2-\underset{CH_3}{CH}-O)_m(CH_2CH_2-O)_nR_7]_2$$

worin s 2 bis 6, $R_8$ Wasserstoff oder Alkyl mit 1 bis 3 Kohlenstoffatomen, t 0 oder 1 ist und m, n und $R_7$ die angegebene Bedeutung haben,

$$-(R_9-\overset{(R_8)_t}{\underset{R_{10}}{N}})_w-R_9-\overset{(R_8)_t}{N}(R_{10})_2$$

worin $R_9$ Alkylen mit 2 bis 8 Kohlenstoffatomen, Cyclohexylen oder Di-($C_1$-$C_3$-Alkylen)-Cyclohexylen, w 0

bis 198, $R_{10}$ Wasserstoff, Alkyl mit 1 bis 3 Kohlenstoffatomen, hydroxysubstituiertes Alkyl mit 2 bis 6 Kohlenstoffatomen, oder

$$-\underset{CH_3}{CH}-CH_2(O\underset{CH_3}{CH}-CH_2)_a(OCH_2CH_2)_b(OCH_2-CH_2)_c\overset{(R_8)_t}{N}H_2$$

ist, worin a 1 bis 20, b 2 bis 50, c 2 bis 20 ist und $R_8$ und t die angegebene Bedeutung haben ;

$R_4$ und $R_5$ unabhängig voneinander Wasserstoff, Alkyl mit 1 bis 5 Kohlenstoffatomen oder

$$-(CH_2-\underset{CH_3}{CH}-O)_m-(CH_2CH_2-O)_n-R_7$$

sind, worin m, n und $R_7$ die angegebene Bedeutung haben, oder

$R_4$ und $R_5$ zusammen mit dem Stickstoffatom, an das sie gebunden sind, einen Morpholinoring bilden ; und

$R_6$ Alkyl mit 1 bis 5 Kohlenstoffatomen, Wasserstoff, wenn wenigstens einer der Substituenten $R_3$, $R_4$ und $R_5$ von Wasserstoff verschieden ist, Alkyl mit 6 bis 23 Kohlenstoffatomen, wenn $R_3$ von Wasserstoff oder Alkyl mit 1 bis 5 Kohlenstoffatomen verschieden ist, oder

$$-(\underset{CH_3}{CH}-CH_2-O)_m-(CH_2CH_2-O)_nR_7$$

ist, worin m, n und $R_7$ die angegebene Bedeutung haben ;

oder $R_4$, $R_5$ und $R_6$ zusammen mit dem Stickstoffatom, an das sie gebunden sind, einen Pyridylring bilden ;

wobei die Summe aller t gleich $^{\oplus}q - 1$ ist.

6. Verbindungen nach Anspruch 5, worin $R_3$—$(CH_2CH_2O)_n$—H ist, worin n 1 bis 10 ist, $R_4$ und $R_5$ unabhängig voneinander Wasserstoff oder —$(CH_2CH_2O)_n$—H sind, worin n 1 bis 10 ist, und $R_6$ Wasserstoff oder —$(CH_2CH_2O)_n$—H ist, worin n 1 bis 10 ist.

7. Verbindungen nach Anspruch 5, worin $R_3$—$(CH_2CH_2O)_n$—H, worin n 1 bis 10 ist, $R_4$—$(CH_2CH_2O)_n$—H n 1 bis 10 ist, $R_5$ Wasserstoff oder —$(CH_2CH_2O)_n$—H, worin n 1 bis 10 ist, und $R_6$ Alkyl mit 6 bis 20 Kohlenstoffatomen ist.

8. Verfahren zur Herstellung der Verbindungen nach Anspruch 1, dadurch gekennzeichnet, dass $q_1$ Mol Säure der Formel

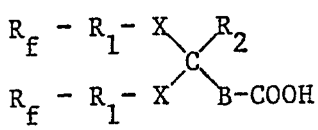

oder des Alkali- oder Erdalkalisalzes dieser Säure, mit $p_1$ Mol Ammoniak oder Amin der Formel Z in Form der freien Base oder eines Salzes in Gegenwart eines inerten Lösungsmittels bei einer Temperatur von 0 bis 100 °C umgesetzt werden.

9. Wässriges Emulsionskonzentrat, das 5 bis 40 Gewichtsprozent des Aminsalzes nach Anspruch 1 enthält.

10. Wässrige Emulsion oder Dispersion zur Oberflächenbehandlung eines cellulosischen oder natürlichen oder synthetischen Polyamid-substrats, um dieses wasser- und ölabweisend zu machen, dadurch gekennzeichnet, dass sie

a) 0,01 bis 5 Gewichtsprozent des Aminsalzes nach Anspruch 1 ;

b) 0 bis 3 Gewichtsprozent Emulgator ;

c) 0 bis 5 Gewichtsprozent Füllstoff, Hydrophobierhilfsmittel, Bakteriostatikum, Farbstoff oder Oberflächenbehandlungsmittel ;

d) 0 bis 10 Gewichtsprozent Schlichtemittel und

e) Wasser

enthält.

11. Wässrige Emulsion zur Massenleimung von Papierpulpe, um diese öl- und wasserabweisend zu machen, dadurch gekennzeichnet, dass diese

a) 0,000 5 bis 0,1 Gewichtsprozent eine Aminsalzes nach Anspruch 1 ;
b) 0,000 01 bis 0,05 Gewichtsprozent Emulgator ;
c) 0 bis 5 Gewichtsprozent Füllstoff, Bakteriostatikum, Fungizid, Farbstoff oder Oberflächenbehandlungsmittel ;
d) 0 bis 10 Gewichtsprozent Leimungsmittel und
e) Wasser

enthält.

12. Verfahren zum Oel- und Wasserabweisendmachen von cellulosischem, natürlichem oder synthetischem Polyamidsubstrat, dadurch gekennzeichnet, dass man das Substrat mit einer wirksamen Menge des wässrigen Emulsion nach Anspruch 10 behandelt und anschliessend trocknet.

13. Verfahren zum Masseverleimen von Papierpulpe, dadurch gekennzeichnet, dass man die Pulpe mit einer wirksamen Menge der wässrigen Emulsion nach Anspruch 11 behandelt, um sie öl- und wasserabweisend zu machen, und die Pulpe anschliessend trocknet.

14. Das gemäss dem Verfahren nach Anspruch 12 öl- und wasserabweisend gemachte cellulosische, natürliche oder synthetische Polyamidsubstrat.

15. Der gemäss dem Verfahren nach Anspruch 13 öl- und wasserabweisend gemachte, aus der Pulpe hergestellte Gegenstand.

**Revendications**

1. Composé ayant la formule

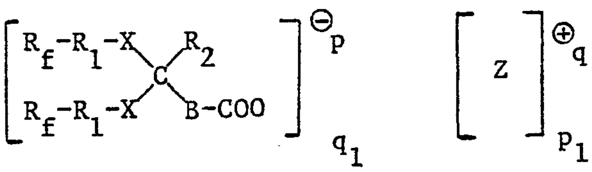

dans laquelle

$R_f$ est un groupe perfluoroalkyle de 4 à 18 atomes de carbone ou un groupe perfluoroalcoxy-perfluoroalkylène de 4 à 18 atomes de carbone ;

$R_1$ est un groupe alkylène de 1 à 12 atomes de carbone, alkylènethioalkylène de 2 à 12 atomes de carbone, alkylèneoxyalkylène de 2 à 12 atomes ou alkylèneiminioalkylène de 2 à 12 atomes de carbone où l'atome d'azote du radical imino contient comme troisième substituant de l'hydrogène ou un reste alkyle de 1 à 6 atomes de carbone ;

X est —S—, —SO— ou —$SO_2$— ;

$R_2$ est de l'hydrogène, un groupe alkyle de 1 à 6 atomes de carbone, phényle, phényl-alkylé ayant jusqu'à 18 atomes de carbone ou $R_2$ est le groupe acide carboxylique —B—COOH ou bien l'anion carboxylate —B—COO— ;

B est une liaison covalente ou un groupe alkylène ayant jusqu'à 18 atomes de carbone, phénylène, phénoxyalkylène de 7 à 14 atomes de carbone, phénylalkylène de 8 à 14 atomes de carbone ou phénylène substitué par un radical alkyle ayant 1 à 4 atomes de carbone ;

Z est le cation ammonium ou amine ;

$^{\ominus}p$ est la charge anionique de l'acide contenant le groupe gem-perfluoroalkyle, et vaut 1 ou 2,

$^{\oplus}q$ est la charge cationique du groupe ammonium ou amine Z et a une valeur de 1 à 200 ; et

l'équivalent molaire cationique $p_1$ est égal à $^{\ominus}p$ et l'équivalent molaire anionique $q_1$ est égal à $^{\oplus}q$, à condition que si $^{\ominus}p$ est 2 et $^{\oplus}q$ est un nombre entier pair, $p_1$ est 1 et $q_1$ est $^{\oplus}q/2$.

2. Composé selon la revendication 1, dans lequel $R_f$ est un groupe perfluoroalkyle de 6 à 14 atomes de carbone ; $R_1$ est un groupe alkylène de 2 à 8 atomes de carbone ; X est —S— ou —SO— ; $R_2$ est de l'hydrogène ou un groupe alkyle de 1 à 4 atomes de carbone ; —B— est une liaison covalente ou un groupe alkylène de 1 à 6 atomes de carbone, $^{\ominus}p$ est 1 et $^{\oplus}q$ est 1 à 4.

3. Composé selon la revendication 1, dans laquelle $R_f$ est un groupe perfluoroalkyle de 6 à 12 atomes de carbone ; $R_1$ est un groupe alkylène de 2 à 4 atomes de carbone ; X est —S— ou —$SO_2$— ; $R_2$ est de l'hydrogène ou un groupe alkyle de 1 ou 2 atomes de carbone ; —B— est une liaison covalente ou un groupe alkylène de 1 à 3 atomes de carbone ; $^{\ominus}p$ est 1 et $^{\ominus}q$ est 1 ou 2.

4. Composé selon la revendication 1, dans lequel Z est le cation de l'ammoniac ou d'une mono- ou polyamine soluble dans l'eau.

5. Composé selon la revendication 4, dans lequel Z est

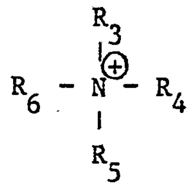

dans laquelle $R_3$ est de l'hydrogène, un groupe alkyle de 1 à 5 atomes de carbone, alkyle de 2 à 6 atomes de carbone substitué par 2 à 6 radicaux hydroxyle, alcényle de 3 à 5 atomes de carbone, cycloalkyle de 5 à 7 atomes de carbone, $—C_1—C_3—$alkylène—COOH,

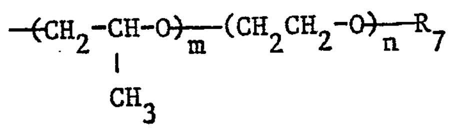

où m est 0 à 6 ; n est 0 à 30, et $R_7$ est de l'hydrogène un groupe alkyle de 1 à 3 atomes de carbone ou alcanoyle de 2 à 4 atomes de carbone, ou bien est :

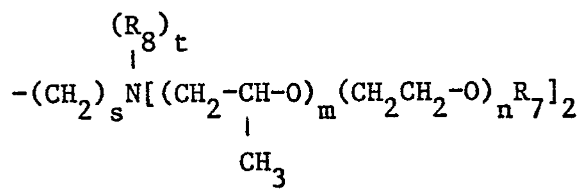

où s est 2 à 6 ; $R_8$ est l'hydrogène ou un groupe alkyle de 1 à 3 atomes de carbone ; t est 0 ou 1, et m, n et $R_7$ ont les définitions données ci-dessus ;

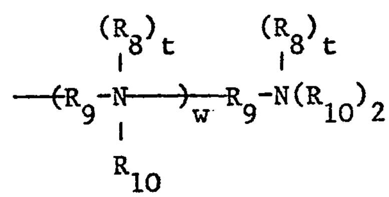

où $R_9$ est un groupe alkylène de 2 à 8 atomes de carbone, cyclohexylène ou di($C_1$ à $C_3$-alkylène)cyclohexylène ; w est 0 à 198 ; $R_{10}$ est de l'hydrogène, un groupe alkyle de 1 à 3 atomes de carbone, alkyle de 2 à 6 atomes de carbone substitués par le radical hydroxyle, ou bien

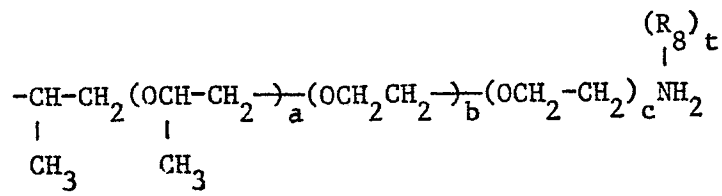

où a est 1 à 20 ; b est 2 à 50 ; c est 2 à 20, et $R_8$ et t ont les définitions ci-dessus ;

$R_4$ et $R_5$ sont indépendamment de l'hydrogène, des groupes alkyle de 1 à 5 atomes de carbone ou

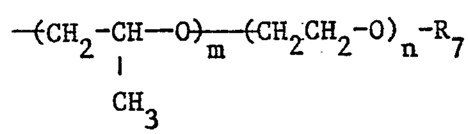

où m, n et $R_7$ ont les définitions ci-dessus ; ou bien

$R_4$ et $R_5$ pris ensemble avec l'atome d'azote auquel ils sont liés représentent un groupe morpholino ; et

$R_6$ est un groupe alkyle de 1 à 5 atomes de carbone ; de l'hydrogène quand au moins un des

symboles $R_3$, $R_4$ et $R_5$ n'est pas de l'hydrogène ; alkyle de 6 à 23 atomes de carbone quand $R_3$ n'est ni de l'hydrogène, ni un groupe alkyle de 1 à 5 atomes de carbone ;

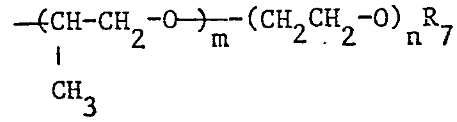

où m, n et $R_7$ ont la définition ci-dessus ;

ou $R_4$, $R_5$ et $R_6$ pris ensemble avec l'atome d'azote auquel ils sont liés représentent un groupe pyridyle, à condition que la valeur totale de t soit égale à ${}^{\oplus}q - 1$.

6. Composition selon la revendication 5, dans lequel $R_3$ est $(CH_2CH_2—C)_n—H$ où n est 1 à 10 ; $R_4$ et $R_5$ sont indépendamment de l'hydrogène ou $(CH_2CH_2—O)_n—H$ où n est 1 à 10, et $R_6$ est de l'hydrogène ou $(CH_2CH_2—O)_n—H$ où n est 1 à 10.

7. Composé selon la revendication 5, dans lequel $R_3$ a la formule $(CH_2CH_2—O)_n—H$ où n est 1 à 10 ; $R_4$ est $(CH_2CH_2—O)_n—H$ où n est 1 à 10 ; $R_5$ est de l'hydrogène ou $(CH_2CH_2—O)_n—H$ où n est 1 à 10, et $R_6$ est un groupe alkyle de 6 à 20 atomes de carbone.

8. Procédé pour préparer le composé selon la revendication 1, en faisant réagir $q_1$ moles de l'acide ayant la formule

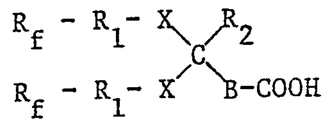

ou du sel de métal alcalin ou de métal alcalino-terreux de cet acide, avec $p_1$ moles d'ammoniac ou d'amine de formule Z, à l'état de sa base libre, ou d'un sel de celle-ci, en présence d'un diluant inerte à une température comprise entre 0 °C et 100 °C.

9. Concentré d'émulsion aqueuse contenant 5 % à 40 % en poids du sel d'amine selon la revendication 1.

10. Emulsion ou dispersion aqueuse pour application topique sur un substrat cellulosique ou un substrat en polyamide naturel ou synthétique, pour rendre le substrat oléophobe et hydrophobe comprenant :

a) 0,01 à 5 % en poids du sel d'amine selon la revendication 1 ;
b) 0 à 3 % en poids d'émulsionnant ;
c) 0 à 5 % en poids de charge, d'auxiliaire hydrofuge, de bactériostat, d'agent colorant ou d'adjuvant conditionnant la surface ;
d) 0 à 10 % en poids d'agent de collage, et
e) l'eau comme restant.

11. Emulsion aqueuse pour le collage interne de la pâte à papier, pour la rendre oléophobe et hydrophobe comprenant :

a) 0,000 5 à 0,1 % en poids d'un sel d'amine selon la revendication 1 ;
b) 0,000 01 à 0,05 % en poids d'émulsionnant ;
c) 0 à 5 % en poids de charge, de bactériostat, de fongicide, d'agent colorant et d'adjuvant conditionnant la surface ;
d) 0 à 10 % d'agent de collage ; et
e) l'eau comme restant.

12. Procédé pour rendre oléophobe et hydrophobe un substrat cellulosique ou un substrat en polyamide naturel ou synthétique comprenant les stades de la mise en contact dudit substrat avec une quantité efficace d'une émulsion aqueuse selon la revendication 10, et le séchage du substrat traité.

13. Procédé pour le collage interne de la pâte à papier comprenant la mise en contact de ladite pâte à papier avec une quantité efficace d'une émulsion aqueuse selon la revendication 11, pour rendre la pâte oléophobe et hydrophobe, puis le séchage de la pâte.

14. Substrat cellulosique, ou substrat en polyamide naturel ou synthétique, hydrophobe et oléophobe obtenu selon le procédé de la revendication 12.

15. Article en pâte à papier oléophobe et hydrophobe obtenu selon le procédé de la revendication 13.